(19) **Europäisches Patentamt / European Patent Office / Office européen des brevets**

(11) **EP 3 996 814 B1**

(12) # EUROPEAN PATENT SPECIFICATION

(45) Date of publication and mention of the grant of the patent:
**17.09.2025 Bulletin 2025/38**

(21) Application number: **20757662.0**

(22) Date of filing: **13.07.2020**

(51) International Patent Classification (IPC):
**A61P 29/00** (2006.01)    **A61K 36/68** (2006.01)
**A61K 36/85** (2006.01)

(52) Cooperative Patent Classification (CPC):
(C-Sets available)
**A61P 29/00; A61K 36/68; A61K 36/85**    (Cont.)

(86) International application number:
**PCT/IB2020/056568**

(87) International publication number:
**WO 2021/005580 (14.01.2021 Gazette 2021/02)**

(54) **USE OF A HERBAL EXTRACT COMPRISING VERBASCOSIDE AND AUCUBIN FOR THE TREATMENT OF OSTEOARTHRITIS OF THE KNEE**

VERWENDUNG EINES KRÄUTEREXTRAKTES ENTHALTEND VERBASCOSID UND AUCUBIN ZUR BEHANDLUNG VON OSTEOARTHRITIS AM KNIE

UTILISATION D'UN EXTRAIT VÉGÉTAL COMPRENANT DU VERBASCOSIDE ET DE L'AUCUBINE POUR LE TRAITEMENT DE L'ARTHROSE DU GENOU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.07.2019 IT 201900011550**
**18.02.2020 IT 202000003287**

(43) Date of publication of application:
**18.05.2022 Bulletin 2022/20**

(73) Proprietor: **Labiotre S.r.l.**
**50028 Barberino Tavarnelle (FI) (IT)**

(72) Inventors:
• **NANNONI, Giulia**
**50028 Barberino Tavarnelle (FI) (IT)**
• **GIUNTINI, Michele**
**50028 Barberino Tavarnelle (FI) (IT)**
• **MARTIGNONI, Ruggero**
**50028 Barberino Tavarnelle (FI) (IT)**

(74) Representative: **Perani & Partners S.p.A.**
**Piazza Armando Diaz, 7**
**20123 Milano (IT)**

(56) References cited:
**WO-A1-2004/069218**    **US-A1- 2005 106 113**

• **SANCHEZ PAUL MAURICIO ET AL: "In vivoanti-inflammatory and anti-ulcerogenic activities of extracts from wild growing andin vitroplants ofCastilleja tenuifloraBenth. (Orobanchaceae)", JOURNAL OF ETHNOPHARMACOLOGY, ELSEVIER IRELAND LTD, IE, vol. 150, no. 3, 18 October 2013 (2013-10-18), pages 1032 - 1037, XP028788973, ISSN: 0378-8741, DOI: 10.1016/ J.JEP.2013.10.002**
• **GEORGIEV M ET AL: "Verbascum xanthophoeniceum-derived phenylethanoid glycosides are potent inhibitors of inflammatory chemokines in dormant and interferon-gamma-stimulated human keratinocytes", JOURNAL OF ETHNOPHARMACOLOGY, ELSEVIER IRELAND LTD, IE, 1 February 2013 (2013-02-01), pages 754 - 760, XP018506085, ISSN: 0378-8741, DOI: 10.1016/J.JEP.2012.10.035**
• **FRANZYK H ET AL: "A caffeoyl phenylethanoid glycoside from Plantago myosuros", PHYTOCHEMISTRY, PERGAMON PRESS, GB, vol. 47, no. 6, 1 January 1998 (1998-01-01), pages 1161 - 1162, XP004293857, ISSN: 0031-9422, DOI: 10.1016/S0031-9422(97)00527-X**

Remarks:
The complete document including Reference
Table(s) and the Sequence Listing(s) can be
downloaded from the EPO website

(52) Cooperative Patent Classification (CPC): (Cont.)

C-Sets
**A61K 36/68, A61K 2300/00;**
**A61K 36/85, A61K 2300/00**

**Description**

The present invention relates to a herbal extract comprising or, alternatively, consisting of an extract of at least one plant of the genus *Aloysia,* preferably *Lippia citriodora* (Palau) Kunth (lemon beebrush) and/or *Verbena Officinalis* L. (common verbena), and an extract of at least one plant of the genus *Plantago,* preferably *Plantago lanceolata* L. (ribwort plantain) and/or *Plantago major* L. (broad-leaved paperbark), comprising, said herbal extract, verbascoside and aucubin. Furthermore, the present invention relates to a composition comprising said herbal extract and the use thereof as anti-inflammatory, analgesic and/or antispasmodic, preferably for use in the treatment of osteoarthritis, such as osteoarthritis of the knee. Lastly, the present invention relates to the method for preparing said herbal extract.

For the treatment of inflammatory and pain conditions (preferably musculoskeletal inflammations, joint inflammations, rheumatic diseases and/or postoperative inflammations), such as for example osteoarthritis in general or osteoarthritis of the knee in particular, mainly available on the market are conventional steroidal anti-inflammatories (i.e. cortisone and derivatives) which have serious adverse effects, and non-steroidal anti-inflammatory drugs (acronym NSAIDs).

The knee is the most commonly affected joint site during symptomatic osteoarthritis (in short, OA). Diagnosed in more than 250 million people worldwide, osteoarthritis of the knee has significant effects on the patient's functionality and it entails considerable social costs in terms of morbidity, for example, loss of work and joint replacement. The results of the osteoarthritis process are cartilage degradation and synovial inflammation; these characteristics are associated with the development of symptoms of pain, stiffness and functional disability.

The current treatment of osteoarthritis, particularly osteoarthritis of the knee, is based on symptom management and primarily on pain control. Clinical guidelines recommend the use of non-steroidal anti-inflammatory drugs (in short, NSAIDs) in patients with persistent symptoms. Although conventional NSAIDs are the most frequently prescribed drugs for osteoarthritis, they are characterised by a number of potential adverse effects, including gastrointestinal bleeding, cardiovascular adverse effects and risk of nephrotoxicity.

NSAIDs more or less reversibly block the binding site of the enzyme cyclooxygenase (COX). Cyclooxygenase is an enzyme involved in the inflammatory process and it leads to the production, starting from arachidonic acid, of numerous inflammatory mediators (i.e. prostaglandins (PG), prostacyclines (PC), thromboxanes (TX) and leukotrienes (LT)). Cyclooxygenase exists in two isoforms: COX-1, a constitutive enzyme of the organism, not involved in the inflammatory process but involved in the production of gastric protection, and COX-2, an inducible enzyme, present during the inflammatory process and responsible for the synthesis of inflammatory mediators in the phlogistic site. The first synthesised NSAIDs, still used today, have no selective action against the two COX isoforms, inhibiting both of them. This explains the anti-inflammatory effect of the inhibition of COX-2 (the enzyme involved in the phlogistic process) and it accounts for the adverse effects at the gastric level, given that the suppression of the COX-1 activity leads to the reduction of the synthesis of prostaglandin (PG) and, thus, of the protective effect of prostaglandins (i.e. PGE2) on the gastric mucosa (PGEs inhibit the production of HCl in the stomach, promote the production of mucus and secretion of bicarbonate, which contrasts gastric acidity). In particular, gastric damage, ulceroid lesions of the mucous membranes, cardiovascular adverse effects and NSAID-induced nephrotoxicity may occur when NSAIDs are used for prolonged or chronic periods.

In order to reduce the adverse effects of NSAIDs, drug research developed selective COX-2 inhibitors with respect to COX-1 (i.e. Celecoxib, etoricoxib, valdecoxib, firocoxib). Due to the high structural similarity between the two isoforms of cyclooxygenase, COX-1 and COX-2, the selectivity of action of these drugs with respect to COX-2 is not complete but only partial, leading to a reduction in gastric adverse effects with respect to non-selective NSAIDs, but not to their complete suppression.

The technical problem addressed and solved by the present invention lies in providing a natural composition with anti-inflammatory effects (preferably for treating musculoskeletal inflammations, joint inflammations, rheumatic diseases and/or postoperative inflammations), analgesic and/or antispasmodic effects, devoid of adverse effects, in particular for the preventive and/or curative treatment of, musculoskeletal inflammation, joint inflammation, rheumatic disease, postoperative inflammations and/or osteoarthritis of the joints, such as knee, hip, shoulder, hand and/or cervical, preferably osteoarthritis of the knee.

Furthermore, the object of the present invention is to provide a composition alternative to the current treatment of osteoarthritis (e.g. NSAIDs), in particular osteoarthritis of the knee, hip, shoulder, hand and/or cervical, preferably osteoarthritis of the knee, which has similar or greater efficacy and does not have the adverse effects that could arise from the treatments currently in use, such as NSAIDs (COX-2 inhibitor NSAIDs).

Following an intense and extensive research activity, the Applicant found that compositions comprising verbascoside and aucubin, as active substances, of natural origin obtained by extracting at least one plant of the genus *Aloysia,* preferably *Lippia citriodora* (lemon beebrush) and/or *Verbena Officinalis* (common verbena), and at least one plant of the genus *Plantago,* preferably *Plantago lanceolata* (ribwort plantain) and/or *Plantago major* (broad-leaved paperbark), are effective as anti-inflammatories (preferably for treating musculoskeletal inflammations, joint inflammations, rheumatic diseases and/or postoperative inflammations), analgesics and antispasmodics. In particular, said herbal compositions or extracts (in short, compositions of the invention and herbal extracts of the invention) act as inhibitors of cyclooxygenase-2 (COX-2), an enzyme involved in inflammatory conditions, selectively with respect to cyclooxygenase-1 (COX-1). In addition, said herbal extracts, or compositions of the invention, comprising or, alternatively, consisting of verbascoside and aucubin (as ingredients or active ingredients) have antioxidant properties.

The study disclosed in "In vivo anti-inflammatory and anti-ulcerogenic activities of extracts from wild growing and in vitro plants of Castilleja tenuiflora Benth (Orobanchaceae)" by Sanchez et al aims at evaluating the cytotoxic, in vivo anti-inflammatory and anti-ulcerogenic activities of extracts from wild growing and in vitro plants of Castilleja tenuiflora Benth [page 1033, left column, last paragraph]. The tested extracts were prepared using whole plant material and extracted in sequence with ethyl acetate, methanol and water, by maceration individually at room temperature for 24 h and subsequently concentrated [chapter 2.2]. Based on the results of Table 1, the content of verbascoside extracted from C. tenuiflora ranges between 1,1% and 26.2%, whilst the content of aucubin ranges between 2,3% and 13,7% by weight on the total weight of the extract (w/w) [chapter 3.1, table 1].

"Verbascum xanthophoeniceum-derived phenylethanoid glycosides are potent inhibitors of inflammatory chemokines in dormant and interferon-gamma-stimulated human keratinocytes" by Georgiev et al is a comparative study of anti-inflammatory effects of *Verbascum xanthophoeniceum* crude extract, its fractions, isolated iridoid glycosides, including aucubin; and phenylethanoid glycosides, including verbascoside [abstract]. In particular, the isolation of the bioactive principles from *V. xanthophoeniceum* brought to the elution of two distinct iridoid and phenylethanoid fractions, with fraction I containing 75% of verbascoside, and fraction II containing 15% forsythoside B [page 756, section 3. Results and discussion, right column]. The Applicant respectfully notes that no information regarding the percentage content of aucubin *in these fractions* is provided.

Although the ability of verbascoside extracted from a plant of the genus *Verbascum* (i.e. *Verbascum xanthophoeniceum*) to inhibit in a dose-dependent manner the gene expression of pro-inflammatory chemocytokines, such as for example IL-8, MCP-1 and IP-10 (Georgiev M et. al, J. of Ethnopharmacology, 1 February 2013, pag. 754-760) is known in literature, its efficacy in the treatment of musculoskeletal inflammations, joint inflammations, rheumatic diseases and/or postoperative inflammations is unknown.

Furthermore, in literature it is known that an extract of *Castilleja tenuiflora* Benth. (*Orobanchaceae*) comprising verbascoside and aucubin has a topical anti-inflammatory effect in an *in vivo* model of *"mouse ear edema"* (Sanchez Paul Mauricio et al., J. of Ethnopharmacology, vol. 150, no. 3, pag. 1032-1037). However, Sanchez's article does not mention anti-inflammatory effects in musculoskeletal inflammations, joint inflammations, rheumatic diseases and/or postoperative inflammations models.

"A caffeoyl phenylethanoid glycoside from Plantago myosuros" by Franzyk et al discloses an extract of Plantago myosuros comprising 0.03% of verbascoside and 0.04% of aucubin [page 1161, section "experimental", right column].

WO2004069218A1 relates to the cosmetic use of verbascoside as an agent for stimulating heat shock protein synthesis and for inhibiting collagenase and elastase and for stimulating hydroxylases by skin cells (claim 1). Preferably, verbascoside is obtained as a herbal extract having a phenyl propanoid glycoside ranging from 0.01% and 75% (claim 7). The herbal extract may be obtained from Lippia citriodora, Verbena officinalis, Plantago major, Plantago media or Plantago lanceolata [page 9, lines 26-29; page 10, lines 9-30; examples 3-4]. Nowhere it is admitted that the extracts obtained from these plants may include also aucubin and that aucubin may eventually have an effect in the stimulation of heat shock proteins or on the inhibition of collagenase/elastase.

US 2005/106113 A1 describes a pharmaceutical composition comprising verbena (e.g. Verbena officinalis) and/or plantain (e.g. Plantago lanceolata), or their respective constituents or extracts [abstract], for the prophylactic or curative topical treatment of dental and periodontal diseases. In Examples 1-3, liquid extract preparation procedures are described from verbena [Example 1], plantain [Example 2], and the combination of verbena and plantain in a 1:1 weight ratio [Example 3], respectively.

Furthermore, the compositions and herbal extracts of the invention are not cytotoxic and they are free from adverse effects, making it possible to administer them, preferably through oral route, to a wide range of subjects, including paediatric subjects, elderly people and pregnant women.

In particular, the compositions and the herbal extracts of the invention are potentially devoid of adverse effects to a greater extent with respect to the NSAIDs currently used in the prior art (COX-2 inhibitor NSAIDs) as they are selective toward COX-2 and have antioxidant properties.

Lastly, the herbal extracts of the invention are easy to prepare (i.e. extraction process) and cost-effective.

These and other objects which will be clear from the detailed description that follows, are attained by the herbal extracts and by the compositions of the present invention due to the technical characteristics claimed in the attached claims.

FIGURES

Figure 1: HPLC chromatogram at a wavelength of A=330 nm for verbascoside in the herbal extract of the invention.

Figure 2: HPLC chromatogram at a wavelength of A=205 nm for aucubin in the herbal extract of the invention.

Figure 3: analysis of COX-2 expression by means of RT-PCR.

Figure 4: PGE2 immunoassay.

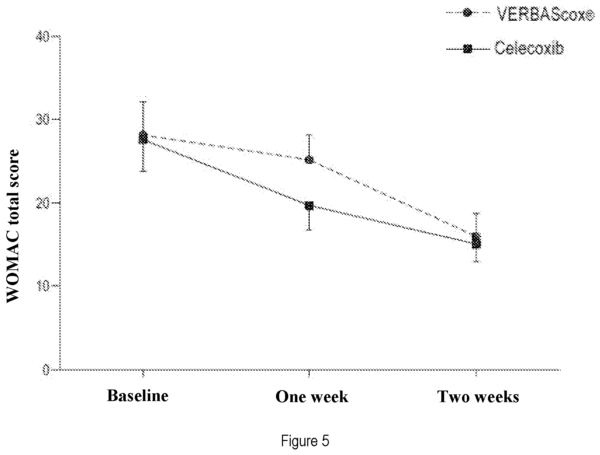

Figure 5: clinical study, comparison of changes in the WOMAC total score in the VERBAScox ® and Celecoxib groups during the 2-week study period. The error bars represent standard deviations.

Figure 6: HPLC chromatogram at a wavelength of A=330 nm for verbascoside in the herbal extract of the invention titrated according to the method reported in the experimental part III.

Figure 7: HPLC chromatogram at a wavelength of A=205 nm for aucubin in the herbal extract of the invention titrated according to the method reported in the experimental part III.

## DETAILED DESCRIPTION OF THE INVENTION

Forming an object of the present invention is a herbal extract (in short, herbal extract of the invention) comprising or, alternatively, consisting of an extract of at least one plant of the genus *Aloysia,* preferably *Lippia citriodora* and/or *Verbena Officinalis,* and an extract of at least one plant of the genus *Plantago,* preferably *Plantago lanceolata* and/or *Plantago major,* wherein said herbal extract comprises - at a percentage by weight - verbascoside comprised in the range from 3% to 10% and aucubin comprised in the range from 0.5% to 15%, wherein said % are with respect to the total weight of the herbal extract.

In a preferred embodiment, said herbal extract of the invention comprising or, alternatively, consisting of an extract of *Lippia citriodora* and/or *Verbena Officinalis* and an extract of *Plantago lanceolata* and/or *Plantago major,* wherein said herbal extract comprises - at a % by weight - verbascoside comprised in the range from 3% to 10% and aucubin comprised in the range from 0.5% to 15%, wherein said % are with respect to the total weight of the herbal extract.

In a more preferred embodiment, said herbal extract of the invention comprising or, alternatively, consisting of an extract of *Lippia citriodora* and an extract of *Plantago lanceolata,* wherein said herbal extract comprises - at a % by weight - verbascoside comprised in the range from 3% to 10% and aucubin comprised in the range from 0.5% to 15%, wherein said % are with respect to the total weight of the herbal extract the herbal extract.

In a preferred embodiment, said herbal extract of the invention (a plant of the genus *Aloysia* and a plant of the genus *Plantago*), preferably *of Lippia citriodora* and *Plantago lanceolata,* comprises - at a percentage by weight - verbascoside comprised in the range from 3% to 10%, more preferably $5 \pm 0.5\%$; and aucubin comprised in the range from 0.5% to 15% preferably from 1% to 5%, more preferably $2 \pm 0.2\%$, wherein said % are with respect to the total weight of the herbal extract; for example, verbascoside from 3% to 10% (*e.g.* 4%, 5%, 6%, 7%, 8% or 9%) and aucubin from 1% to 5% (*e.g.* 2%, 3% or 4%)), or verbascoside $5 \pm 0.5\%$ and aucubin $2 \pm 0.2\%$.

For example, said herbal extract of the invention comprises verbascoside and aucubin at percentage by weight ratio with respect to the weight of said herbal extract [% w/w verbascoside : % w/w aucubin] comprised from 10:1 to 1:1, preferably from 5:1 to 1:1, more preferably from 3:1 to 1:1 (for example about 2.5:1).

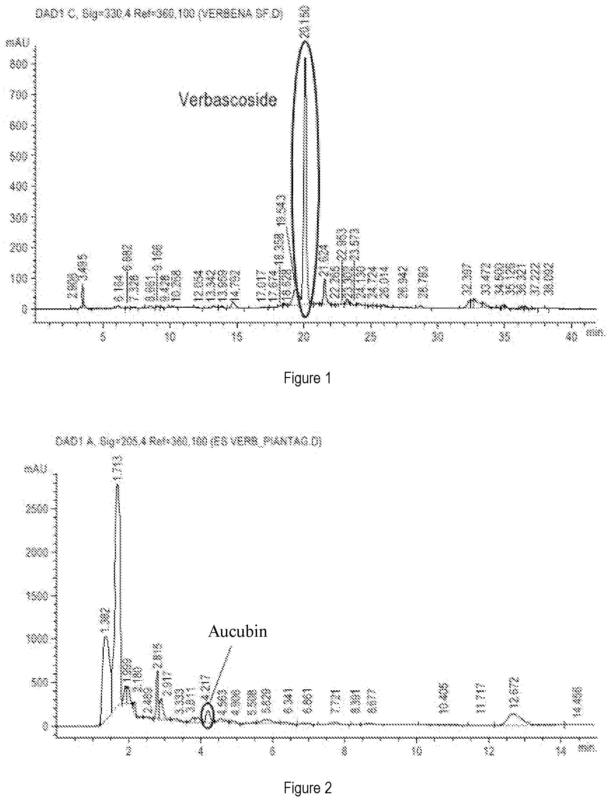

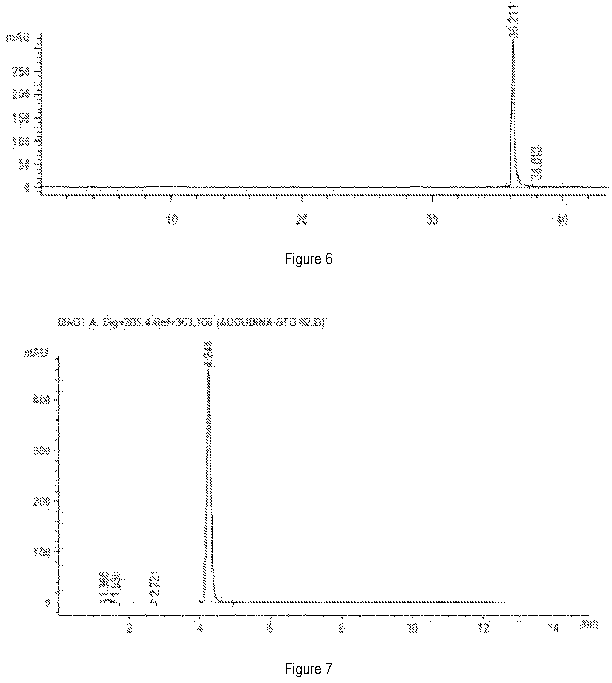

The content of verbascoside and aucubin in the herbal extract of the invention - at a percentage by weight with respect to the total weight of the extract - can be determined according to techniques known to the man skilled in the art; preferably they can be determined by means of such quality/quantitative techniques of high pressure or high performance liquid chromatography (HPLC) with detection by means of DAD (diode-array detector) at a wavelength of A=330 nm for verbascoside and λ=205 nm for aucubin (Figure 1 and Figure 2) and/or according to the titration methods reported in the experimental part (Figure 6 and Figure 7).

In the context of the present invention, the expression *"Lippia citriodora"* is used to indicate *Lippia citriodora* (Palau) Kunth or *Aloysia citriodora* Palau, commonly called "lemon beebrush" or *"lemon verben". Lippia citriodora* is a perennial shrub plant of the genus *Aloysia* of the family *Verbenaceae* of the order *Lamiales,* not to be confused with the "common verbena" or *Verbena officinalis.* Furthermore, synonyms of *Lippia citriodora* are *Aloysia triphylla, Verbena citriodora and Verbena triphyilla.* The phytocomposition of *Lippia citriodora* is mainly represented by terpenoids, but there are also various polyphenols including verbascoside.

In the context of the present invention, the expression "Verbena *officinalis"* is used to indicate *Verbena officinalis* L. (L. such as Carl Nilsson Linnaeus), perennial shrub plant of the genus *Aloysia* of the family *Verbenaceae* of the order *Lamiales,* commonly called "verbena" or "common verbena".

Verbascoside (IUPAC name [6-[2-(3,4-dihydroxyphenyl)ethoxy]-5-hydroxy-2-(hydroxymethyl)-4-(3,4,5-trihydroxy-6-methyl oxan-2-yl)oxy oxan-3-yl] (E)-3-(3,4-dihydroxyphenyl)prop-2-enoatel), CAS N° 61276-17-3) is a phenylpropanoid widespread in the plant kingdom, characterised by a residue of caffeic acid and of hydroxytyrosol bound to a glucopyranoside on the opposite sides of the sugar chain respectively through an ester and glyosidic bond. It can be found in species of the order of *Lamiales,* or it can also be produced in plant cell cultures. Verbascoside (also known as acteoside) is a secondary metabolite produced by the plant (*e.g. Lippia citriodora* or *Verbena officinalis*) as a defence agent against parasites and insects.

In the context of the present invention, the expression *"Plantago lanceolata"* is used to indicate *Plantago lanceolata L.* (1753) (L. such as Carl Nilsson Linnaeus), commonly called "narrowleaf plantain" or "ribwort plantain". *Plantago lanceolata* is a perennial medicinal herb plant of the genus *Plantago* of the family of *Plantaginaceae* of the order of *Lamiales.*

In the context of the present invention, the expression "Plantago *major"* is used to indicate *Plantago major* L.(1753) (L. such as Carl Nilsson Linnaeus), a perennial medicinal herb plant of the genus *Plantago* of the family of *Plantaginaceae* of the order *of Lamiales. Plantago major* is known by many common names: broadleaf plantain, white man's foot, common plantain, greater plantain, plantain, ribgrass, ribwort, buckhorn, dog's tongue, lamb's tongue.

Aucubin (IUPAC name (2S,3R,4S,5S,6R)-2-[[(1S,4aR,5S,7aS)-5-hydroxy-7-(hydroxymethyl)-1,4a,5,7a-tetrahydrocyclopenta[c]pyran-1-yl]oxy]-6-(hydroxymethyl)oxane-3,4,5-triol, CAS N° 479-98-1) is found in various glycoside plants. Iridoids belong to the largest class of terpenoids.

The herbal extract of the invention can be an extract of leaves, roots, flowers, stems or stigmas of said at least one plant of the genus *Aloysia,* preferably *Lippia citriodora* and/or *Verbena officinalis* and an extract of leaves, roots, flowers, stems or stigmas of said at least one plant of the genus *Plantago,* preferably *Plantago lanceolata* and/or *Plantago major,* preferably it is an extract of *Lippia citriodora* and/or *Verbena officinalis* and of leaves of *Plantago lanceolata* and/or *Plantago major;* more preferably it is an extract of leaves of *Lippia citriodora* and of leaves of *Plantago lanceolata.*

Preferably, said herbal extract of the invention, preferably *of Lippia citriodora* and *Plantago lanceolata,* comprising verbascoside and aucubin - at a percentage by weight - of the invention (preferably verbascoside from 3% to 10%, and aucubin from 0.5%, to 15%, more preferably from 1% to 5%, as reported in detail in the present invention), is a dry extract having a dry residue at a percentage by weight comprised in the range from 90% to 100% with respect to the total weight of the herbal extract, preferably from 95% to 100%, more preferably from 98% to 100%. Advantageously, the herbal extract of the invention is a dry extract of leaves of *Lippia citriodora* and/or *Verbena officinalis,* preferably *Lippia citriodora,* and of leaves of *Plantago lanceolata* and/or *Plantago major,* preferably *Plantago lanceolata,* comprising verbascoside and aucubin at the % by weight of the invention, preferably verbascoside titrated to 5 ± 0.5% and aucubin titrated to 2 ± 0.2%.

In a particularly preferred embodiment, said herbal extract of the invention (according to any one of the embodiments and aspects described) is a dry extract in granular form (or granular dry extract).

The granules (or microgranules) of said dry extract in granular form are granules of almost uniform dimensions and having an average particle size comprised in the range from 20 mesh to 150 mesh, preferably from 20 mesh to 100 mesh, more preferably from 20 mesh to 50 mesh, measured through mechanical or manual sieve shakers.

Besides the extract of said at least one plant of the genus *Aloysia,* preferably *Lippia citriodora* and/or *Verbena officinalis,* and the extract of said at least one plant of the genus Plantago, preferably *Plantago lanceolata* and/or *Plantago major,* said dry extract in granular form advantageously further comprises a carrier selected from the group comprising or, alternatively, consisting of: maltodextrin, gum arabic, inulin, vegetable fibres, polyalcohols, fructooligosaccharides, lecithins, dextrose, isomalt and sugars of various kinds; preferably said carrier is maltodextrin.

Advantageously, said dry extract in granular form comprises said carrier (preferably maltodextrin) at a percentage by weight comprised from 5% to 80%, preferably from 10% to 60%, more preferably from 20% to 50%, for example about 25%, 30%, 35%, 45%.

A preferred example of the herbal composition or extract of the invention, obtained according to the process of the present invention, is a dry extract in granular form comprising a carrier (e.g. maltodextrin), wherein said extract is an extract of leaves of *Lippia citriodora* and of leaves of *Plantago lanceolata,* and wherein said extract comprises verbascoside from 3% to 10% *(e.g.* 5 ± 0.5%), and aucubin from 0.5%, to 15%, preferably from 1% to 5% *(e.g.* 2 ± 0.2%) wherein the percentages are percentages by weight with respect to the total weight of the dry extract in granular form.

Said dry extract in granular form, preferably comprising said carrier, has the advantage of being highly soluble and, furthermore, highly flowable, allowing a better performance of industrial machinery for the processing thereof, for example for the preparation of compositions, such as for example capsules, tablets, powders for sachets and liquid compositions, comprising the herbal extract of the invention.

Furthermore, the dry extract in granular form of the invention, preferably comprising said carrier, has the advantage of being formulated in mouth-soluble and water-soluble form, i.e. in easily usable administration forms.

In a further embodiment, the dry extract in granular form of the invention can be covered or encapsulated or coated with a coating matrix. wherein said coating matrix is such to confer to the dry extract in granular form both a gastro-protection and a modulated release of the active ingredients contained therein (i.e. verbascoside and aucubin) into the intestine. Said coating matrix (coating agent) may comprise or, alternatively, consist of free fatty acids or relative esters, waxes, lacquers or mixtures thereof. The expression gastro-protection coating matrix is used to indicate that the granules of dry extract are coated with a casing which protects them from the acidic environment of the stomach so that they arrive unaltered in the intestine where they release the active ingredient allowing the absorption thereof.

Forming an object of the present invention is a composition comprising or, alternatively, consisting of the herbal extract of the invention according to any one of the embodiments described in the present invention and, optionally, at least one food or pharmaceutical grade additive and/or excipient.

Should said additive and/or excipients not be present, the composition of the invention coincides with the herbal extract of the invention, preferably with the dry herbal extract in granular form of the invention comprising said carrier.

In the context of the present invention, said at least one pharmaceutical or food grade additive and/or excipient, optionally present in the composition of the invention, is a substance devoid of therapeutic activity suitable for pharmaceutical or food use. Said additives and/or excipients acceptable for pharmaceutical or food use comprise all the auxiliary substances known to the man skilled in the art such as, for example, diluents, solvents (including water, glycerine, ethyl alcohol), solubilisers, thickeners, sweeteners, anti-caking agents, fillers, flavour enhancers, dyes, lubricants, surfactants, antimicrobials, antioxidants, preservatives, pH stabilising buffers and mixtures thereof; specific examples are: phosphate buffers, magnesium stearate, silicon dioxide, cross-linked sodium carboxymethyl cellulose, microcrystalline cellulose, hydroxypropyl methyl cellulose, natural or artificial flavours.

In an embodiment the composition of the invention, comprising the herbal extract of the invention and, optionally, at least one food or pharmaceutical grade additive and/or excipient as defined in the present description, may further comprise a further component (or active component) selected from the group comprising or, alternatively, consisting of: vitamins of group B, C, D, E, organic and/or inorganic salts of magnesium, of selenium, of zinc and mixtures thereof.

Forming an object of the present invention is a pharmaceutical composition, nutraceutical composition, dietary supplement product or a food product or a food for special medical purpose (FSMP or Novel food), a medical device composition or a cosmetic composition comprising or, alternatively, consisting of the composition of the invention according to the embodiments described in the present description.

In the context of the present invention, the expression "medical device" is used in the meaning according to the Italian Legislative Decree n° 46 dated 24 February 1997 or according to the new Medical Device Regulation (EU) 2017/745 (MDR).

The composition of the present invention may be in liquid form, such as a solution, two-phase liquid system, suspension, dispersion or syrup, in semi-solid form, such as gel, cream or foam, or in solid form, such as powder, granules or microgranules, flakes, aggregates, tablet, chewable tablet, capsule, lozenge, pill, bar and equivalent forms.

The composition of the invention can be formulated for oral (oral use or gastroenteric administration), sublingual (or buccal), transmucosal or topical administration.

In a preferred embodiment, the composition of the invention is for oral use, preferably in solid form; even more preferably in a form of granules, microgranules, flakes, powder, mouth-soluble or soluble powder or granules or microgranules, tablet, capsule or soft-gel.

Forming an object of the present invention is the composition or herbal extract of the invention as described above (according to any embodiment or aspect described), wherein said composition is for use as a medicament.

Said herbal extract or said composition according to the invention is for use as anti-inflammatory, analgesic and/or antispasmodic in a needy subject, preferably by oral administration.

In particular, the composition or herbal extract of the invention is for use in the symptomatic treatment of inflammatory musculoskeletal diseases, rheumatoid diseases, inflammatory joint diseases and inflammatory postoperative diseases, preferably for use in methods for the treatment of osteoarthritis, rheumatoid arthritis and ankylosing spondylitis.

Advantageously, the composition or herbal extract of the invention is for use in the preventive and/or curative treatment of osteoarthritis of the knee, hip, shoulder, hand and/or cervical, preferably for osteoarthritis of the knee.

In particular, the composition or herbal extract of the invention for use as an analgesic, as a substance which attenuates or eliminates the sensation of pain without reducing the state of consciousness, is a composition for use in the treatment of toothache, dysmenorrhea, postoperative pain, cervical pain, arthritic pain, abdominal pain or cramps, musculoskeletal pain, postpartum pain, sports-related trauma or contusion pain and general pain.

The herbal extract of the invention titrated in verbascoside and aucubin exerts significant anti-inflammatory and analgesic effects acting as a specific inhibitor of COX-2; furthermore, **it** is not cytotoxic and is devoid of relevant adverse effects. Said activity of the herbal extract of the invention can be attributed to a synergistic or mutual enhancement effect carried out by verbascoside and aucubin against the overexpression of COX-2 in inflammatory conditions or in pain conditions.

In addition, the composition or herbal extract of the invention is for use as an antioxidant, antimicrobial and antipyretic for lowering body temperature in case of fever.

In an alternative embodiment, the composition or herbal extract of the invention is for use as a coadjuvant to a preventive and/or curative anti-inflammatory treatment (preferably musculoskeletal inflammations, joint inflammations, rheumatic diseases and/or postoperative inflammations), analgesic, antispasmodic, antioxidant, antimicrobial and/or antipyretic, in particular for the diseases, symptoms and/or disorders described in the present invention, preferably osteoarthritis, such as osteoarthritis of the knee, hip, shoulder, hand and/or cervical.

The composition or herbal extract of the invention shall be deemed indistinctly for human or veterinarian use, i.e. as composition to be applied to human or animal subjects by means of the methods known to the man skilled in the art.

The present invention also relates to a method for the preventive and/or symptomatic treatment of inflammatory musculoskeletal, rheumatological, joint and postoperative diseases (e.g. osteoarthritis, rheumatoid arthritis and ankylosing spondylitis) and symptoms and/or disorders related therewith, preferably osteoarthritis, such as osteoarthritis of the knee, hip, shoulder, hand and/or cervical; to a preventive and/or symptomatic, analgesic and/or antispasmodic treatment method to reduce or eliminate symptoms and/or disorders related with or arising from pain (e.g. toothache, dysmenorrhea, postoperative pain, cervical pain, arthritic pain, abdominal pain or cramps, musculoskeletal pain, postpartum pain, sports-related trauma or contusion pain and general pain), such as a treatment method; to an antioxidant, antimicrobial and/or antipyretic treatment method, wherein said treatment methods comprise administering the composition of the invention as defined above at a therapeutically effective amount to a needy subject (amount defined based on knowledge of the man skilled in the art).

The composition of the invention for use in the preventive or curative treatment of osteoarthritis of the knee, hip, shoulder, hand and/or cervical, preferably of the knee, can be administered through oral route to the needy subject at an amount such to provide from 400 mg/day to 1600 mg/day, preferably from 500 mg/day to 1200 mg/day, more preferably from 600 mg/day to 1000 mg/day, of said herbal extract (preferably dry extract in granular form comprising a carrier) comprising or, alternatively, consisting of an extract of *Lippia citriodora* (Palau) Kunth and/or *Verbena Officinalis* L, and an extract of *Plantago lanceolata* L. and/or *Plantago major* L., wherein said herbal extract comprises verbascoside at a percentage by weight comprised in the range from 3% to 10%, and aucubin at a percentage by weight comprised in the range from 0.5% to 15%, preferably from 1% to 5%, wherein said percentages are with respect to the total weight of the herbal extract (or any percentage reported in the described embodiments).

Advantageously, the composition of the invention for use in the preventive and/or curative treatment of osteoarthritis of the knee, hip, shoulder, hand and/or cervical, preferably of the knee, can be administered orally to the needy subject at an amount such to provide from 100 mg/day to 200 mg/day, from 100 mg/day or 400 mg/day to 1600 mg/day, preferably from 500 mg/day to 1200 mg/day, more preferably from 600 mg/day to 1000 mg/day, of said herbal extract (preferably dry extract in granular form comprising a carrier) comprising or, alternatively, consisting of an extract of *Lippia citriodora* (Palau) Kunth and an extract of *Plantago lanceolata* L., wherein said extract of herbs comprises verbascoside at a percentage by weight comprised in the range from 3% to 10% *(e.g.* $5\pm0.5\%$), and aucubin at a percentage by weight comprised in the range from 1% to 5% *(e.g.* $2 \pm 0.5\%$) wherein the percentages are with respect to the total weight of the herbal extract.

The present invention also relates to the non-therapeutic use of the composition of the invention according to the various embodiments reported in the present description, wherein said use is for a non-therapeutic treatment of malaise or discomfort sensations related to the sphere of pain and spasms of the intestine and abdomen.

Lastly, forming an object of the present invention is a method for the preparation of the herbal extract of the invention (in short, method of the invention), according to any one of the embodiments reported in the present description, comprising the steps of:

(I) preparing (a) at least one plant of the genus *Aloysia* comprising verbascoside, preferably *Lippia citriodora* and/or *Verbena Officinalis,* and/or (b) at least one plant of the genus *Plantago* comprising aucubin, preferably *Plantago lanceolata* and/or *Plantago major,* in an extraction plant; followed by

(II) mixing (a) and/or (b) with a hydroalcoholic solvent (c), preferably ethanol/water, wherein the [(a) and/or (b)]:(c) by weight ratio is comprised in the range from 1:1 to 1:25, preferably from 1:3 to 1:20, more preferably from 1:6 to 1:15; followed by

(III) heating, under stirring, at a temperature comprised in the range from 40°C to 80°C, preferably from 50°C to 70°C, for a period of time comprised in the range from 20 minutes to 240 minutes, preferably from 30 minutes to 180 minutes, more preferably from 40 minutes to 120 minutes, to obtain a crude extract (d) comprising a solid phase and a liquid phase; followed by

(IV) separating said liquid phase of the crude extract (d) from the solid phase of the crude extract (d) to obtain an isolated liquid phase (e);

(V) concentrating the isolated liquid phase (e).

The method of the invention, comprising steps (I) to (V), can be carried out by extracting (a), preferably *Lippia citriodora,* and (b), preferably *Plantago lanceolata,* simultaneously on a single extraction plant. Alternatively, the extraction of (a), preferably *Lippia citriodora,* and the extraction of (b), preferably *Plantago lanceolata,* can be carried out separately on two different extraction plants.

In a preferred embodiment, (a), preferably *Lippia citriodora,* and (b), preferably *Plantago lanceolate,* are extracted simultaneously on a single extraction plant.

In said method of the invention, the extraction (i.e. step (II) and step (III)) is carried out in a short time allowing to extract the active substances (i.e. verbascoside and/or aucubin) from the raw material extracted leaving the components of the phytocomplex originally contained in plants (a) and (b) as defined in the context of the present invention, preferably (a) *Lippia citriodora* and (b) *Plantago lanceolata* unaltered and balanced.

The method of the invention comprises steps (I) to (V) described and, steps (VI) to (VII) and optionally step (VIII),

- the step (V) of concentrating the isolated liquid phase (e) is carried out by partially removing the solvent (c) for example for evaporation, until obtaining a soft extract (f) having a dry residue comprised in the range from 20% to 80% by weight on the total weight of the soft extract (f), preferably from 30% to 70%, more preferably from 40% to 60%; and
- the concentrating step (V) is followed by the step (VI) of drying said soft extract (f) to obtain a dry extract (g), wherein said dry extract (g) has a dry residue at a % by weight comprised in the range from 90% to 100% with respect to the total weight of the dry extract (g), preferably from 95% to 100%, more preferably from 98% to 100%.

Said drying step (VI) comprises the partial or almost complete removal of the solvent (c) used during extraction, for example by evaporating the solvent using techniques conventional to the man skilled in the art.

The dry extract (g) obtained by the method of the invention (steps (I) to (VI)) is the herbal extract comprising or, alternatively, consisting of an extract of at least one plant of the genus *Aloysia*, preferably *Lippia citriodora* and/or *Verbena Officinalis*, and an extract of at least one plant of the genus *Plantago*, preferably *Plantago lanceolata* and *Plantago major*, comprises - at a percentage by weight - verbascoside comprised in the range from 3% to 10% and aucubin comprised in the range from 0.5% to 15%, wherein said percentages are with respect to the total weight of the herbal extract, in the various embodiments described in the present description.

In an embodiment, the drying step (VI) is followed by the step (VII) of granulating said dry extract (g), to obtain a dry extract in granular form (h) as defined in the context of the present invention.

The drying step (VI) is concurrent with the granulating step (VII) given that said drying step (VI) is carried out by means of a fluid-bed granulator, according to standard procedures known to the man skilled in the art, wherein a carrier is used and said carrier is mixed with said soft extract (f), to obtain a granulated dry extract (h) as defined in the context of the present invention.

Advantageously, said carrier is selected from the group comprising or, alternatively, consisting of maltodextrin, gum arabic, inulin, vegetable fibers, polyalcohols, fructooligosaccharides, lecithins, dextrose, isomalt and sugars of various kinds, preferably maltodextrin.

The steps of drying and granulating with granulation method by means of a fluid-bed granulator leads to the obtainment of a dry herbal extract in the form of granules according to the present invention having almost uniform dimensions, wherein said granules have the following characteristics:

- very high flowability which allows excellent processability, for example the production of capsules and tablets of uniform weight and hardness, with a minimum loss and greater productivity in the oral solids production step;
- dissolution rate which is much higher with respect to conventional dry powder extracts; this is reflected in a higher *in vivo* bioavailability and a better pharmacokinetics of the active substances of the extract (*i.e.* verbascoside and aucubin);
- absence of powder formation upon opening the sachet package (e.g. soluble or mouth-soluble compositions);
- possible coating of the granular extract with a suitable agent to obtain controlled release granules or specific pH-resistant granules (*e.g.* gastroprotected).

Advantageously, in the method of the invention, steps (I) to (V) or (VI) or (VII) are followed by the step (VIII) of determining the verbascoside and aucubin content in the extract (f), (g) or (h) by means of techniques such as high-pressure or high-performance liquid chromatography (HPLC) quality/quantitative techniques at a wavelength of A=330 nm for verbascoside and A=205 nm for aucubin.

In a preferred embodiment, in the method of the invention comprising steps (I) to (V) or (VI) or (VII) or (VIII), in said preparation step (I) the leaves of (a), preferably *Lippia citriodora*, comprising verbascoside and/or leaves of (b), preferably *Plantago lanceolata*, comprising aucubin; preferably the dried leaves of (a), preferably *Lippia citriodora*, and/or dried leaves of (b), preferably *Plantago lanceolata*; more preferably wherein said dried leaves are in a form selected from powder, tisane form or filter form.

In a preferred embodiment, in the method of the invention comprising steps (I) to (V) or (VI) or (VII) or (VIII), in said step (II) the hydroalcoholic solvent (c) is an ethanol/water mixture having an alcohol volume comprised in the range from 10° to 80° alcohol, preferably from 20° to 70° alcohol.

Advantageously, in the method of the invention comprising steps (I) to (V) or (VI) or (VII) or (VIII), the separating step (IV) is carried out by means of a technique selected from the techniques of clarification, decantation, centrifugation and/or filtration of the substrate to be purified, preferably centrifugation.

Advantageously, in the method of the invention comprising steps (I) to (V) or (VI) or (VII) or (VIII), the concentrating step (V), as the step of removing or evaporating part of the solvent used during extraction, is carried out according to a vacuum concentration technique selected from vacuum with bubble evaporator, vacuum with falling film and vacuum with thin layer, preferably with thin layer.

The concentration of the vacuum extract allows to considerably reduce the production time and to recover the solvent.

In a more preferred embodiment, the method of the invention comprises or, alternatively, consists of the steps of:

- (I) preparing the leaves of (a) *Lippia citriodora* comprising verbascoside and the leaves of (b) *Plantago* lanceolata comprising aucubin; preferably dried leaves; followed by
- (II) mixing (a) and (b) with the ethanol/water solvent (c) having an alcohol volume comprised in the range from 10° to 80° alcohol, wherein (a) and (b) are at a by weight ratio comprised in the range from 1:6 to 1:15; followed by
- (III) heating, under stirring, at a temperature comprised in the range from 50°C to 70°C, for a period of time comprised in the range from 40 minutes from 120 minutes, to obtain a crude extract (d) comprising a solid phase and a liquid phase; followed by
- (IV) separating the liquid phase of the crude extract (d) from the solid phase of said crude extract (d), preferably with the centrifugation technique, to obtain an isolated liquid phase (e); followed by
- (V) concentrating the isolated liquid phase (e) by partially evaporating the solvent (c) until obtaining a soft extract (f) having a dry residue comprised in the range from 40% to 60% by weight on the total weight of the soft extract (f); followed by
- (VI) drying said soft extract (f) to obtain a dry extract (g), wherein said dry extract (g) has a dry residue at a % by weight comprised in the range from 90% to 100%, preferably from 98% to 100%, with respect to the total weight of the dry extract (g); wherein said drying step (VI) is carried out by means of a fluid-bed granulator wherein a carrier is used and said carrier is mixed with said soft extract (f); and wherein said carrier is selected from the group comprising or, alternatively, consisting of maltodextrin, gum arabic, inulin, vegetable fibers, polyalcohols, fructooligosaccharides, lecithins, dextrose, isomalt and sugars of various kinds, preferably maltodextrin.

For the sake of clarity, with the aim of attaining the object of the present invention, the components (or active components) of the herbal extract of the present invention, such as the extract of (a) and the extract of (b) titrated in verbascoside and aucubin according to the percentages by weight described in the present invention, may also be administered separately (preferably at a time range from 30 minutes to 60 minutes) and in any order but, preferably, the extract of (a) and the extract of (b) are administered to a subject simultaneously, even more preferably in a single composition so as to obtain a more rapid effect and for ease of administration.

When the components (or active components) of the herbal extract of the present invention, such as the extract of *Lippia citriodora* and the extract of *Plantago lanceolata* titrated in verbascoside and aucubin are administered in a single composition, said single composition corresponds to the composition of the present invention.

Unless specified otherwise, the expression composition or extract or other comprising a component at an amount "comprised in a range from x to y" is used to indicate that said component can be present in the composition or extract or other at all the amounts present in said range, even though not specified, extremes of the range comprised.

EXPERIMENTAL PART I

Since COX-2 is an inducible enzyme involved in inflammation, the effect of a composition according to the invention (herbal extract of *Lippia citriodora* and *Plantago lanceolata* titrated in verbascoside (>= 5%) and aucubin (>= 2%)) toward COX-2 expressions in human neutrophils stimulated by LPS (bacterial lipopolysaccharide) using both reverse transcription of the polymerase chain reaction (in short RT-PCR) and a PGE2 immunoassay was studied.

METHODS AND MATERIALS

I.1. Isolation of neutrophils

Human neutrophils were isolated from whole blood samples taken from 10 healthy volunteers (5 men and 5 women, mean age: 34.1 $\pm$ 3.7 years) using the EasySep™ Direct human neutrophil isolation kit (STEMCELL Technologies, Vancouver, Canada). After isolation, neutrophils were resuspended at a concentration of 5 * 106 cells/ml in Hank's balanced saline solution (37°C) containing 10 mM of HEPES pH 7.4, 1.6 mM $Ca^{2+}$ and not $Mg^{2+}$.

I.2. Material

- LPS was purchased from InvivoGen (Toulouse, France).

- The plant extract (water/ethanol extraction) in granular form according to the invention (comprising plant extract of *Lippia citriodora* and *Plantago lanceolata,* and maltodextrin as carrier) titrated in verbascoside (from 5% to 10% by weight with respect to the weight of the plant extract) and aucubin (from 2% to 5% by weight with respect to the weight of the plant extract) (VERBAScox®, registered trademark of LABIOTRE S.R.L., Barberino Tavarnelle, Italy). For example, an 800 mg VERBAScox ® tablet comprises 240mg of maltodextrin and 560mg of plant extract (of *Lippia citriodora* and *Plantago lanceolata*) titrated in verbascoside (from 5% to 10% by weight with respect to the total weight of the extract) and aucubin (from 2% to 5% by weight with respect to the total weight of the extract).

- Celecoxib (a known pharmacological inhibitor of COX-2) was purchased from Sigma-Aldrich (St. Louis, MO, USA).

I.3. Cell viability

Cell viability was determined using the MTT test kit (Promega, Madison, WI, USA) according to the manufacturer's protocol (MTT: 3-(4,5-dimethylthiazol-2-yl)-2,5-diphenyltetrazolium bromide). In order to determine the cytotoxicity of the herbal extract of the invention, the cells were incubated with aqueous solutions of the herbal extract of the invention at three different concentrations (1%, 3% and 5%) for 24 hours. The cultures of the control group were not treated and the viability thereof was set to 100%. For comparison purposes, the cells were also treated for 24 hours with a Celecoxib 1% solution, which was prepared as described above [Agrawal S., et al.: Solubility enhancement of poorly water-soluble Celecoxib for parenteral formulations. Int J Pharm Sei (2012) 3: 2325-2336]. After incubation, 10 $\mu$l of the MTT labeling reagent was added to each well. After 4 hours, a 100 $\mu$l aliquot of solubilisation solution was added, followed by incubation for 12 hours. Absorbance was subsequently measured with a microplate reader (Bio-Tek, Winooski, VT, USA) at a test wavelength of 595 nm and a reference wavelength of 690 nm. The optical density (OD) was calculated as the difference between absorbance at reference wavelength and absorbance at test wavelength. The viability percentage was calculated using the following formula: (OD of treated sample / control OD) x 100.

I.4. Incubations with LPS under various experimental conditions

Experiments were conducted on RPMI medium (RPMI: Rosewell Park Memorial Institute, cation-deficient medium Ca2+ and Mg2+ commonly used for white blood line cells) supplemented with 1% fetal bovine serum (37°C, 5% $CO_2$, 95% humidity) after seeding neutrophils (5x10$^5$ cells/well) in a 96-well plate. The following conditions were applied:

1) untreated cells (control),
2) cells exposed to 5 $\mu$g/mL of LPS for 24 hours,
3) cells exposed to 5 $\mu$g/mL of LPS plus herbal extract of the invention in 1% solution for 24 hours,
4) cells exposed to 5 $\mu$g/ml of LPS plus herbal extract of the invention in a 3% solution for 24 hours,
5) cells exposed to 5 $\mu$g/ml of LPS plus herbal extract of the invention in 5% solution for 24 hours and
6) cells exposed to 5 $\mu$g/ml of LPS plus Celecoxib in 1% solution for 24 hours.

I.5. Analysis of expression of COX-1 and COX-2 mRNA

RT-PCR was performed to identify mRNA expressions of COX-1 and COX-2. Total RNA was isolated from neutrophils using RNAzol™B (TEL-TEST, Friendswood, TX, USA). 2 $\mu$L of RNA and 2 $\mu$L of random hexamers (Promega) were added together and the mixture was heated to 65°C for 10 minutes. 1 $\mu$L of AMV reverse transcriptase (Promega), 5 $\mu$L of 10 mM dNTP (Promega), 1 $\mu$L of RNasin (Promega) and 5 $\mu$L of 10 x AMV RT buffer (Promega) were then added to the mixture. The final volume was brought to 50 $\mu$L using diethyl pyrocarbonate-treated water and the reaction mixture was then incubated at 42°C for 1 hour. PCR amplification was conducted in a reaction volume of 40 $\mu$L containing 1 $\mu$L of appropriate cDNA, 1 $\mu$L of each primer set at a concentration of 10 pM, 4 $\mu$L of 10 x RT buffer, 1 $\mu$l of 2.5 mm dNTP and 2 units of Taq DNA polymerase (Promega).

The primer sequence for human COX-1 was as follows: 5'- CGCCAGTGAATCCCTGTTGTT-3' and reverse 5'-AAGGTGGCATTGACAAACTCC-3'.

The primer sequence for human COX-2 was as follows: 5'- CTGGCGCTCAGCCATACAG-3' and reverse 5'-CGCACTTATACTGGTCAAATCCC-3'.

The primer sequence for glyceraldehyde 3-phosphate dehydrogenase (GAPDH; *gene housekeeping*) was as follows: 5'-GGAGCGAGATCCCTCCAAAAT-3' and reverse 5'-GGCTGTTGTCATACTTCTCATGG-3'.

PCR amplifications were conducted using a GeneAmp® 9600 PCR (Perkin Elmer, Norwalk, CT, USA) under the following conditions: initial denaturation at 94°C for 5 minutes, followed by 40 cycles, each consisting of denaturation at 94°C for 30 seconds, annealing at 58°C for 30 seconds, extension at 72°C for 30 seconds, with a final extension step at the end of the procedure at 72°C for 5 minutes. The quantities of RT-PCR products for each of the mRNA species under the six different experimental conditions described above were densitometrically calculated using Molecular Analyst™ software (Bio-Rad, Hercules, CA, USA). Experiments were conducted in triplicate and quantitative data of gene expression were normalised to GAPDH expression levels.

I.6. Evaluation of PGE2 concentrations

Evaluation of PGE2 concentrations in each well under six different experimental conditions was conducted using a commercially available competitive PGE2 enzyme immunoassay kit (GE Healthcare, Milwaukee, WI, USA). All tests were conducted three times.

I.7. Data analysis

Statistical calculations were performed using GraphPad 7.0 (GraphPad Inc., San Diego, CA, USA). The results are expressed as means$\pm$ standard deviations. The data were analysed using a remote ANOVA followed by post-hoc Dunn test. A two-tailed P value <0.05 was considered statistically significant.

RESULTS

I.8. Cell viability

Based on the MTT test results, the viability of the cells incubated for 24 hours with the herbal extract of the invention solubilised at 1%, 3% and 5% of the concentrations was 91 $\pm$ 3%, 87 $\pm$ 5% and 81 $\pm$ 7% of the control value (100%) observed in the untreated cells. The cell viability of cells incubated with Celecoxib 1% was 90 $\pm$ 2%.
Overall, the MTT test results revealed that the herbal extract of the invention did not exert any significant cytotoxicity in human neutrophils.

I.9. Analysis of COX-1 and COX-2 expression by means of RT-PCR

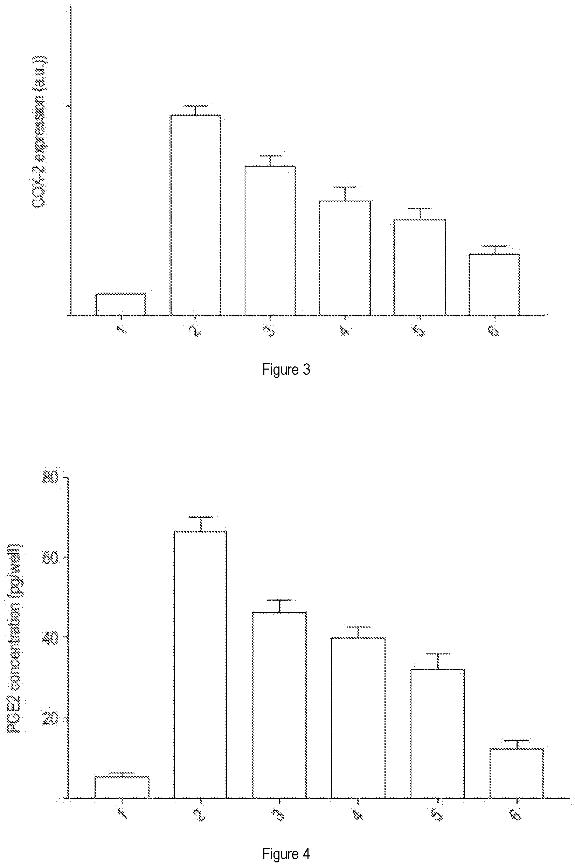

RT-PCR analysis of COX-1 and COX-2 mRNA levels was conducted in order to provide an estimate of the relative expression levels of these genes under the six different experimental conditions outlined above. COX-1 and COX-2 mRNA levels in untreated cells were used as control value and set to 100 arbitrary units (a.u.).
After treatment with 5 $\mu$g/ml of LPS for 24 hours, the COX-1 expression was 108 $\pm$ 10 a.u.
COX-1 mRNA levels after exposure to 5 $\mu$g/ml of LPS plus herbal extract of the invention at concentrations of 1%, 3% and 5% for 24 hours were 111 $\pm$ 8, 96 $\pm$ 11 and 91 $\pm$ 9 a.u., respectively.
Expression levels of cells exposed to 5 $\mu$g/ml of LPS plus Celecoxib 1% for 24 hours were 116 $\pm$ 4 a.u. All of these changes were not statistically significant, suggesting that treatments do not affect COX-1 expression.
COX-2 mRNA level after treatment with 5 $\mu$g/ml of LPS for 24 hours was significantly increased to 971 $\pm$ 49 a.u.
COX-2 mRNA levels after exposure to 5 $\mu$g/ml of LPS plus herbal extract of the invention at concentrations of 1%, 3% and 5% for 24 hours were 702 $\pm$ 51, 536 $\pm$ 63 and 449 $\pm$ 46 a.u., respectively (all P <0.001 versus treatment with 5 $\mu$g/ml of LPS).
Expression levels of cells exposed to 5 $\mu$g/ml of LPS plus Celecoxib 1% for 24 hours were 283 $\pm$ 37 a.u. (Figure 3; P <0.001 toward herbal extract of the invention at a concentration of 5%).

I.10. PGE2 Immunoassay

The results of the PGE2 immunoassay revealed that, after 24 hours of exposure to LPS, the amount of PGE2 increased from 5.12 $\pm$ 1.24 pg/well to 66.21 $\pm$ 3.67 pg/well.
PGE2 levels after exposure to 5 $\mu$g/ml of LPS plus herbal extract of the invention at concentrations of 1%, 3% and 5% for 24 hours were 46.29 $\pm$ 3.10 pg/ well, 39.83 $\pm$ 2.97 pg/ well and 32.11 $\pm$ 3.89 pg/ well (all P <0.001 toward treatment with 5 $\mu$g/ml of LPS).
PGE2 concentrations at 5 $\mu$g/ml of LPS plus Celecoxib 1% for 24 hours were 19.21 $\pm$ 2.39 pg/well (Figure 4; p <0.001 toward herbal extract of the invention at the concentration of 5%).

DISCUSSION OF THE RESULTS

Increased expression of COX-2 leads to increased production of PGE2, which in turn is closely linked with the onset of inflammation and pain. The main results of these areas of study are as follows:

1) the herbal extract of the invention titrated in verbascoside and aucubin is not significantly cytotoxic as shown by the MTT assay;

2) the extract does not significantly inhibit COX-1, while it is capable of suppressing LPS-induced COX-2 overexpression at mRNA level in human neutrophils;

3) In an *in vitro* inflammatory experimental model, the effect of the herbal extract of the invention at a concentration of 5% was comparable to that obtained with Celecoxib 1%. However, Celecoxib significantly outperformed the herbal extract of the invention in terms of absolute and relative reduction of COX-2 mRNA expression and PGE2 production in human neutrophils.

These results demonstrate that the herbal extract of the invention is capable of inhibiting both COX-2 expression and PGE2 synthesis in a cell type (human neutrophils) that plays a key role in acute inflammation. In other words, these results demonstrate that the herbal extract of the invention titrated in verbascoside and aucubin is safe (non-cytotoxic) and it can exert significant anti-inflammatory and analgesic effects by acting as a specific inhibitor of COX-2.

Consequently, the experimental data of the present description demonstrate that verbascoside and aucubin contained in the herbal extract of the invention exert synergistic or mutual enhancement effects against the expression of COX-2 in inflammatory conditions or in pain conditions.

EXPERIMENTAL PART II

The Applicant has conducted a clinical study: randomized, single-blind, active-controlled pilot study in order to study the clinical efficacy of oral administration of VERBAScox®, a plant extract according to the invention capable of inhibiting human cyclooxygenase-2 (COX-2), in patients with mild to moderate osteoarthritis of the knee (in short, OA).

The main object of this pilot study was to compare the extent of pain relief and improved functional capacity of patients subjected to oral administration of VERBAScox® compared to patients treated with Celecoxib, a known pharmaceutical inhibitor of COX-2. In addition, serum levels of substance P (in short, SP), a neuropeptide belonging to the family of tachykinins involved in pain perception, as a biochemical marker of treatment response, were measured.

METHODS AND MATERIALS

II.1. Patients

This clinical study was a randomised, single-blind, active-controlled pilot study in 150 consecutive patients (117 women and 33 men). Patients aged 40 to 75 years with clinical diagnosis of osteoarthritis of the knee according to American College of Rheumatology [16] criteria were eligible. The inclusion criteria were as follows: 1) presence of symptoms for at least one month, 2) number of leukocytes in synovial fluid < 2000/ml, 3) pain on a visual analogue scale (VAS) $\geq$ 2 at rest and 4) duration of stiffness in the morning $\leq$ 30 min. The following exclusion criteria were applied: 1) pregnancy or breastfeeding, 2) history of severe renal, hepatic, cardiac, gastrointestinal or haematological diseases, 3) presence of malignant tumours, 4) presence of neurological or psychiatric disorders, 5) atopy or allergic disorders, 6) diabetes mellitus or other endocrine disorders, 7) coagulation disorders, 8) history of peptic ulcer disease, 9) use of corticosteroid in the four weeks prior to the study and 10) use of NSAIDs in the two weeks prior to the study. According to the Kellgren-Lawrence classification system [17], 61.3% and 38.7% of patients in the study had scores of 1 and 2, respectively. Patients were then classified as having mild to moderate osteoarthritis of the knee. The study protocol was in line with the principles of the Helsinki Declaration. Prior to the study, each patient was informed of the purpose of the study and each was given written informed consent.

II.2. Materials

- VERBAScox® (composition according to the invention): as indicated in point I.2; in the present clinical study it was in the form of tablets containing 800 mg including 240mg of maltodextrin and 560mg of plant extract (of *Lippia citriodora* and *Plantago lanceolata*) titrated in verbascoside (from 5% to 10% by weight with respect to the total weight of the extract) and aucubin (from 2% to 5% by weight with respect to the total weight of the extract).
- Celecoxib (comparative composition): oral capsules of Celecoxib 200 mg, marketed by Pfizer (New York, NY, United States).

II.3. Procedures

The study period was two weeks.

At baseline, body mass index (BMI) and duration of pain were recorded for all participants.

Patients (n = 150) were randomised into three study groups (1:1:1 ratio). The randomisation sequence was generated by a special software.

Patients in the control group (n = 50) were not subjected to any treatment (watchful waiting).

Patients in the VERBAScox® group (n = 50) received oral administration (800 mg/day; one tablet) of VERBAScox® plant extract for 2 weeks.

Patients in the Celecoxib group (n = 50) who took Celecoxib (200 mg/day; one capsule) for 2 weeks.

The medical examiners and laboratory staff were blind to the treatment group, while the study patients were not blind to the treatment assigned. All participants were asked to discontinue other concomitant therapies during the study period.

A total of three assessments were performed (at baseline, one week and at the end of the 2-week treatment period).

II.4. Clinical endpoints

Clinical endpoints included:

1) patient's evaluation of the arthritis pain score (VAS) at rest (range: 0-10; where 0 is no pain and 10 is maximum pain),
2) patient's evaluation of the arthritis pain score (VAS) at movement (range: 0-10; where 0 is no pain and 10 is maximum pain),
3) joint motion range (degrees) and
4) WOMAC (Western Ontario and McMaster University) index score [Salaffi et al. Osteoarthritis cartilage 2003; 11: 551-60].

WOMAC score consists of sub-scales that measure pain (range: 0-20; where 0 is no pain and 20 is the maximum pain), stiffness (range: 0-8, where 0 is no stiffness and 8 is the maximum stiffness) and physical functionality (range: 0-68, where 0 is the best functionality and 68 is the worst functionality). The resulting total composite score ranges from 0 to 96.

II.5. Measurements of serum levels of substance P

At each evaluation, samples of venous blood for serum separation were collected in test tubes. The blood was left to clot at room temperature for 30 minutes and then centrifuged at $1000 \times rpm$ for 15 minutes. The serum was removed and the aliquots were frozen at -80°C until the time of laboratory measurements. Serum levels of substance P were evaluated using an ELISA test according to the manufacturer's protocol (R&D Systems, Minneapolis, MN, USA). Absorbance at 450 nm was measured on an ELISA microplate reader (PerkinElmer, Waltham, MA, USA). The detection limit of this assay was 25 pg/ml and the coefficients of intra and inter-assay variation were 8% and 13%, respectively. All samples were processed simultaneously at the end of the study by blinded laboratory personnel compared to clinical data.

II.6. Safety

Safety was evaluated by recording treatment-emergent adverse events and changes with respect to the baseline in clinical laboratory tests, in vital signs and in objective examinations, all of which were performed during visits at weeks 1 and 2. Clinically relevant changes in laboratory values were defined as:

- aspartate aminotransferase (AST) and/or alanine transaminase (ALT) $\geq 3 \times$ upper limit of normal (ULN),
- creatinine $\geq 1.3 \times$ ULN,
- blood urea nitrogen (BUN) $\geq 2 \times$ ULN,
- decrease in haematocrit value $\geq 5$ percentage points with respect to the baseline and
- decrease in haemoglobin value $\geq 2$ g/dl from baseline.

II.7. Statistical analysis

Categorical variables are expressed as counts and percentage frequencies and were compared using chi-square tests. The continuous variables are reported as means $\pm$ standard deviation and they were compared at the three evaluation points using ANOVA followed by post-hoc Newman-Keuls tests. All analyses were conducted using GraphPad Prism, version 7.0 (GraphPad Inc., San Diego, CA, USA) and SPSS 17.0 (SPSS Inc., Chicago, IL, USA). Two-tailed P values <0.05 were considered statistically significant.

RESULTS

II.8. Patients - Study Groups

The baseline characteristics of the three study groups are shown in Table 1. There were no significant intergroup differences in age, gender, BMI, pain duration, pain at rest, pain at motion, range of motion (degrees), and WOMAC scores. Baseline safety laboratory parameters (AST, ALT, creatinine, BUN, haematocrit and haemoglobin) all were within the normal range (data not shown). The study sample may therefore be considered representative of a clinical population of patients with mild to moderate osteoarthritis of the knee requiring pharmacological treatment.

II.9. Clinical endpoints

No patients withdrew from the study. The clinical endpoints of the three study groups are shown in Table 2. No significant differences over time were observed in the control arm (watchful waiting). With respect to the baseline values, no significant difference was evident between the VERBAScox® group and the Celecoxib group at the end of the 2-week study period.

Figure 1 compares changes in the WOMAC total score in the VERBAScox® and Celecoxib groups during the 2-week study period.

II.10. Serum levels of substance P

Table 3 summarises the temporal changes in serum substance P levels in the three study groups. No significant differences over time were observed in the control arm (watchful waiting). At one week, Celecoxib produced a statistically significant reduction in serum P levels compared to baseline values. However, no significant difference was evident between the VERBAScox® group and the Celecoxib group at the end of the 2-week study period and both treatment arms showed similar reductions with respect to baseline values.

II.11. Safety

No treatment-emergent adverse events occurred in the group treated with VERBAScox® (Table 4).

DISCUSSION

In this randomised, single-blind, active-controlled pilot study, it was found that administration of VERBAScox® (800 mg/day) in patients with mild to moderate osteoarthritis of the knee, is effective and safe at least as a standard therapeutic dose of Celecoxib in terms of pain relief and improvement of functional capacity after a 2-week treatment cycle.

It is interesting to note that the time course of the reduction of substance P in serum followed a similar trend, suggesting that the clinical effects of VERBAScox® and Celecoxib could at least partly be mediated by a decrease in serum levels of this biochemical pain and inflammation mediator.

Administration of VERBAScox® for two weeks was safe, with treatment-emergent adverse events sporadic and similar to those observed in the Celecoxib group; none of these events resulted in discontinuation of treatment. In particular, there were no changes with respect to the baseline in laboratory safety tests in both VERBAScox® and Celecoxib groups.

The efficacy of the analgesic effect of Celecoxib in osteoarthritis of the knee occurs within two days of initiation of treatment and it is known that 200 mg/day intake provides effective control of painful symptoms. This dose was then used for the active control treatment arm.

Through *in vitro* experiments (see experimental part I), it was previously demonstrated that VERBAScox® is capable of inhibiting COX-2 in a dose-dependent manner. In the present study, the comparable clinical efficacy of VERBAScox® and Celecoxib was demonstrated by similar scores in VAS at rest, VAS at motion, range of motion and WOMAC total score at 2 weeks.

VERBAScox® can be clinically effective against osteoarthritis, particularly osteoarthritis of the knee, from mild to moderate not only due to ability to inhibit COX-2, but also due to other active properties of the components thereof. As a matter of fact. verbascoside is characterised by a high antioxidant power, while aucubin can attenuate inflammatory responses induced by tumour necrosis factor a.

The possibility that VERBAScox® may exert further antioxidant and anti-inflammatory effects independent of COX-2 would explain why its clinical efficacy was similar to that of Celecoxib at 2 weeks, despite being a less potent inhibitor of COX-2. It is important to note that the magnitude of reductions in the WOMAC total score at the end of the study (from 28.2 ± 4.0 to 16.0 ± 2.7 in the VERBAScox®group and from 27.6 ± 3.8 to 15.1 ± 2.2 in the Celecoxib group) should be considered a clinically relevant improvement. In this regard, it should be borne in mind that the clinically important minimum differences in the WOMAC total score are comprised between 9.5 and 10.1 for osteoarthritis of the knee and of the hip.

In the present study, improvements in clinical symptoms in both active treatment groups (VERBAScox® and Celecoxib) were accompanied by a significant reduction in serum levels of substance P, a neuropeptide released from sensory nerves which exerts several pro-inflammatory effects. It should be noted that substance P is not only involved in the perception of pain, but it is also capable of upregulating the expression of COX-2. In recent years, more and more evidence has shown the role of substance P in human joint diseases, including osteoarthritis. It is therefore possible that the reduction in substance P levels may contribute to the anti-inflammatory and pain-relieving effects of VERBAScox® and Celecoxib.

In conclusion, the results obtained in the present clinical study demonstrate that oral administration of VERBAScox® (800 mg/day, composition according to the invention) in patients with mild to moderate osteoarthritis of the knee, is effective and safe in terms of pain relief and improvement of functional capacity after a 2-week treatment period.

Furthermore, the results obtained in the present clinical study demonstrate that oral administration of VERBAScox® (composition according to the invention) and treatment with Celecoxib (NSAIDs, comparative composition of the prior art) for 2 weeks have similar effects in reducing the symptoms of mild to moderate osteoarthritis of the knee.

The reduction in serum levels of substance P followed an overlapping time pattern and could be at least partly responsible for the observed clinical effects.

Table 1 (Characteristics of the three baseline study groups)

| Variable | No treatment (n = 50) | Administration of VERBAScox® 800 mg/day (n = 50) | Treatment with Celecoxib 200 mg/day (n = 50) | P |
|---|---|---|---|---|
| Age, years | 57.1 ± 5.3 | 56.8 ± 5.6 | 57.4 ± 5.5 | 0.31 |
| Women/ men | 39/11 | 40/10 | 38/12 | 0.89 |
| Body mass index, kg/m$^2$ | 25.7 ± 2.4 | 25.8 ± 2.2 | 26.0 ± 2.5 | 0.48 |
| Duration of pain, months | 3.1 ± 0.4 | 2.9 ± 0.6 | 3.2 ± 0.5 | 0.51 |
| Pain at rest, VAS (0-10) | 2.6 ± 0.3 | 2.7 ± 0.4 | 2.6 ± 0.4 | 0.59 |
| Pain at motion, VAS (0-10) | 3.7 ± 0.6 | 3.9 ± 0.7 | 3.8 ± 0.6 | 0.79 |
| Range of motion (degrees) | 133 ± 10 | 132 ± 9 | 134 ± 10 | 0.81 |
| *WOMAC scores* | | | | |
| Pain | 5.6 ± 0.6 | 5.8 ± 0.8 | 5.7 ± 0.8 | 0.73 |
| Stiffness | 1.4 ± 0.3 | 1.5 ± 0.4 | 1.4 ± 0.3 | 0.68 |
| Physical functionality | 20.1 ± 2.4 | 20.9 ± 3.6 | 20.5 ± 3.2 | 0.75 |
| Total | 27.1 ± 3.3 | 28.2 ± 4.0 | 27.6 ± 3.8 | 0.59 |
| Abbreviations: VAS, visual analogue scale; WOMAC, Western Ontario and McMaster Universities. | | | | |

Table 2 (time course of clinical endpoints in the three study groups;

| The data are expressed as means and standard deviations. *P < 0.001 with respect to the baseline. †P < 0.001 with respect to the VERBAScox® arm at one week. | | | | | | | | | |
|---|---|---|---|---|---|---|---|---|---|
| | No treatment (n = 50) | | | Administration of VERBAScox® 800 mg/day (n = 50) | | | Treatment with Celecoxib 200 mg/day (n = 50) | | |
| | *Baseline* | *One week* | *End of study* | *Baseline* | *One week* | *End of study* | *Baseline* | *One week* | *End of study* |
| Pain at rest, VAS (0-10) | 2.8 ± 0.3 | 2.8 ± 0.2 | 2.5 ± 0.3 | 2.7 ± 0.4 | 2.5 ± 0.7 | 1.7 ± 0.5* | 2.9 ± 0.4 | 2.0 ± 0.3*,† | 1.5 ± 0.4 * |
| Pain at motion, VAS (0-10) | 3.7 ± 0.6 | 3.9 ± 0.8 | 3.8 ± 0.7 | 3.9 ± 0.7 | 3.6 ± 0.9 | 2.4 ± 0.6* | 3.8 ± 0.6 | 2.9 ± 0.5*,† | 2.2 ± 0.4* |
| Range of motion (degrees) | 133 ± 10 | 135 ± 13 | 131 ± 12 | 132 ± 9 | 141 ± 18 | 168 ± 21* | 134 ± 10 | 160 ± 15*,† | 173 ± 19* |
| *WOMAC scores* | | | | | | | | | |
| Pain | 5.6 ± 0.6 | 5.8 ± 0.7 | 5.5 ± 0.9 | 5.8 ± 0.8 | 5.2 ± 1.4 | 3.3 ± 0.9* | 5.7 ± 0.8 | 4.1 ± 0.9*,† | 3.0 ± 0.9* |
| Stiffness | 1.4 ± 0.3 | 1.6 ± 0.3 | 1.5 ± 0.2 | 1.5 ± 0.4 | 1.3 ± 0.4 | 0.8 ± 0.3* | 1.4 ± 0.3 | 1.0 ± 0.2*,† | 0.7 ± 0.1* |
| Physical functionality | 20.1 ± 2.4 | 22.5 ± 2.7 | 21.8 ± 2.6 | 20.9 ± 3.6 | 18.7 ± 2.5 | 11.9 ± 2.0* | 20.5 ± 3.2 | 14.6 ± 2.1*,† | 11.4 ± 1.7* |
| Total | 27.1 ± 3.3 | 29.9 ± 3.8 | 28.8 ± 3.6 | 28.2 ± 4.0 | 25.2 ± 3.1 | 16.0 ± 2.7* | 27.6 ± 3.8 | 19.7 ± 2.9*,† | 15.1 ± 2.2 |

Table 3 (Time course of serum substance P levels in the three study groups)

| | No treatment (n = 50) | | | Administration of VERBAScox® 800 mg/day (n = 50) | | | Treatment with Celecoxib 200 mg/day (n = 50) | | |
|---|---|---|---|---|---|---|---|---|---|
| | Baseline | One week | End of study | Baseline | One week | End of study | Baseline | One week | End of study |
| SP, pg/ml | 423 ± 85 | 451 ± 96 | 444 ± 72 | 445 ± 91 | 423 ± 80 | 312 ± 77* | 436 ± 95 | 358 ± 78*, † | 299 ± 64* |
| *P < 0.001 with respect to the baseline. †P < 0.001 with respect to the VERBAScox® arm at one week. SP: substance P | | | | | | | | | |

Table 4 (Treatment-emergent adverse events observed in the VERBAScox® and Celecoxib arms)

| | Administration of VERBAScox® 800 mg/day (n = 50) | Treatment with Celecoxib 200 mg/day (n = 50) |
|---|---|---|
| *Gastrointestinal adverse events* | | |
| | | |
| Nausea | 1 (2%) | 2 (4%) |
| Constipation | 0 (0%) | 2 (4%) |
| | | |

(continued)

| Other adverse events | | |
|---|---|---|
| Headache | 2 (4%) | 3 (6%) |
| | | |
| Itching | 0 (0%) | 1 (2%) |

The data are expressed as the number of patients (percentages in brackets).

EXPERIMENTAL PART III

1. METHOD of TITRATION of verbascoside via HPLC in the plant extract according to the invention (Figure 6).

SAMPLE PREPARATION

1 g of sample is extracted with 50 ml of EtOH 70° for 30 minutes by ultrasound. It is centrifuged and the same extraction is repeated on the residue. The solution is brought to volume in a 100 ml flask; the solution is filtered with a 0.45 $\mu$m filter and injected by HPLC.

CHROMATOGRAPHIC CONDITIONS

Column: Zorbax Rapid Resolution SB C18 4.6 X 250 mm with particles of 5 $\mu$m diameter + Zorbax Reliance Cartridge guard column.

 Wavelength: 330 nm

 Flow: 0.8 ml/min

 Mobile phase:

- A = 0.2 % aqueous solution of phosphoric acid
- B = acetonitrile

Elution scheme:

- A/B 88/12 → 85/15 in 4 minutes
- A/B 85/15 → in isocratic for 5 minutes
- A/B 85/15 → 75/25 in 10 minutes
- A/B 75/25 → in isocratic for 8 minutes
- A/B 75/25 → 0/100 in 10 minutes
- A/B 0/100 → in isocratic for 5 minutes

STANDARD CALIBRATION LINE (TABLE 5)

Table 5

| Verbascoside | | |
|---|---|---|
| Concentration | Area | Calibration line |
| 0.98 mg/ml<br>0.49 mg/ml<br>0.245 mg/ml | 21802<br>11275.5<br>5456.62158 | P = 0.00004<br>I = 0.00023<br>r = 0.99979 |

(continued)

| Verbascoside | | |
| --- | --- | --- |
| Concentration | Area | Calibration line |
| 0.098 mg/ml | 2068.52881 | |

Solubilisation solvent of the standard: Methanol from HPLC.

At each analysis, inject a standard at known concentration and verify that the resulting area has a value of $\pm$ 10% compared to the values of the areas used for the calibration line.

CALCULATIONS

The percentage content of verbascoside is calculated with the following formula:

$$\% \, verbascoside = \frac{(A_{camp} * P + I) * V}{(p * 10)}$$

Where:

- $A_{camp}$ = verbascoside area present in the sample;
- P = slope of the calibration line;
- I = intercept of the calibration line;
- V = total volume of the extract in ml;
- p = weight of the sample in g.

2. METHOD of TITRATION of aucubin via HPLC in the plant extract according to the invention (Figure 7)

SAMPLE PREPARATION

0.5 g of sample are extracted with 50 ml of MeOH for 30 minutes by ultrasound. It is centrifuged and the same extraction is repeated on the residue. The combined extracts are evaporated under reduced pressure at a temperature below 40°C. The residue is taken up with 10 ml of MeOH 70° and transferred to a centrifuge test tube containing 2.5 g of alumina. The mixture is stirred for a few minutes and centrifuged. The supernatant is decanted into a 250 ml flask, while on the precipitate two further washings are carried out with 40 ml MeOH 70° each. The combined supernatants are concentrated under reduced pressure at a temperature below 40°C up to a volume of about 15 ml. The concentrated liquid is transferred to a 25 ml flask. The flask is washed first with 1 ml of $CH_3CN$ and then with 5 ml of $H_2O$. The washing liquids are poured into the flask and brought to volume with $H_2O$. The solution is filtered with a 0.45 $\mu$m filter and injected by HPLC.

CHROMATOGRAPHIC CONDITIONS

Column: Spherisorb ODS-2 C18 250x4.0mm with 5$\mu$m diameter particles+ Zorbax Reliance Cartridge guard column.

Wavelength: 205 nm
Flow: 1 ml/min
Mobile phase:

- A = $H_2O$
- B = acetonitrile

Elution scheme:

- A/B 95/5 isocratic for 15 min

STANDARD CALIBRATION LINE (TABLE 6)

Table 6

| Aucubin | | | |
|---|---|---|---|
| | Concentration | Area | Calibration line |
| | 0.58 mg/ml | 7289.81006 | P = 0.00008 |
| | 0.29 mg/ml | 3787.33276 | I = -0.00798 |
| | 0.058 mg/ml | 781.43475 | r = 0.99980 |

Solubilisation solvent of the standard: $H_2O$ from HPLC.

At each analysis, inject a standard at known concentration and verify that the resulting area has a value of $\pm$ 10% compared to the values of the areas used for the calibration line.

CALCULATIONS

The percentage content of aucubin is calculated with the following formula:

$$\% \, aucubina = \frac{(A_{camp} * P + I) * V}{(p * 10)}$$

Where:

- $A_{camp}$ = aucubin area present in the sample;
- P = slope of the calibration line;
- I = intercept of the calibration line;
- V = total volume of the extract in ml;
- p = weight of the sample in g.

**Claims**

1. A herbal extract comprising or, alternatively, consisting of an extract of at least one plant of the genus *Aloysia* and an extract of at least one plant of the genus *Plantago,* wherein said herbal extract comprises verbascoside in the range from 3% to 10% and aucubin in the range from 0.5% to 15%, wherein said percentages are percentages by weight with respect to the weight of said herbal extract.

2. The herbal extract according to claim 1, wherein said at least one plant of the genus *Aloysia* is selected from the group comprising or, alternatively, consisting of: *Lippia citriodora* (Palau) Kunth, *Verbena Officinalis* L. and a mixture thereof; and/or wherein said at least one plant of the genus *Plantago* is selected from the group comprising or, alternatively, consisting of: *Plantago lanceolata* L., *Plantago major* L. and a mixture thereof.

3. The herbal extract according to claim 1 or 2, wherein said at least one plant of the genus *Aloysia* is *Lippia citriodora* (Palau) Kunth and wherein said at least one plant of the genus *Plantago* is *Plantago lanceolata* L.

4. The herbal extract according to any one of claims 1 to 3, wherein said herbal extract comprises verbascoside in the range from 3% to 10% and aucubin in the range from 1% to 5%, wherein said percentages are percentages by weight with respect to the weight of said herbal extract; preferably wherein said herbal extract is a dry extract in granular form having a dry residue at a percentage by weight comprised in the range from 90% to 100% with respect to the total weight of the dry extract.

5. A composition comprising or, alternatively, consisting of: said herbal extract according to any one of claims 1 to 4, and at least one food or pharmaceutical grade additive and/or excipient.

6. The herbal extract according to any one of claims 1 to 4 or the composition according to claim 5 for use as a medicament.

7. The herbal extract according to any one of claims 1 to 4 or the composition according to claim 5 for use in the symptomatic treatment of inflammatory musculoskeletal diseases, rheumatoid diseases, inflammatory joint diseases and inflammatory postoperative diseases, preferably for use in methods for the treatment of osteoarthritis, rheumatoid arthritis and ankylosing spondylitis.

8. The herbal extract or the composition for use according to claim 6 or 7, wherein said herbal extract or composition is administered through oral route.

9. The herbal extract or the composition for use according to claim 7 or 8, wherein said herbal extract or composition is for use in the preventive and/or curative treatment of osteoarthritis of the knee, hip, shoulder, hand and/or cervical.

10. The herbal extract or the composition for use according to claim 9, wherein said herbal extract or said composition is for use in the preventive and/or curative treatment of osteoarthritis of the knee.

11. The herbal extract according to any one of claims 1 to 4 or the composition according to claim 5 for use as an analgesic and/or antispasmoic medicament, by administering a therapeutically effective amount of said herbal extract or composition to a needy subject, preferably through oral route.

12. The herbal extract or the composition for use according to claim 11, wherein said herbal extract or said composition is for use in a method for the treatment of toothache, dysmenorrhea, postoperative pain, cervical pain, arthritic pain, abdominal pain or cramps, musculoskeletal pain, postpartum pain, sports-related trauma or contusion pain and/or general pain.

13. The herbal extract or the composition for use according to any one of claims 7 to 12, wherein said herbal extract or said composition is administered through oral route to the needy subject at an amount such to provide said herbal extract at an amount comprised in the range from 400 mg/day to 1600 mg/day, preferably from 500 mg/day to 1200 mg/day, more preferably from 600 mg/day to 1000 mg/day.

14. A method for preparing the herbal extract according to any one of claims 1 to 4 comprising the steps of:

(I) preparing - in an extraction plant - (a) at least one plant of the genus Aloysia comprising verbascoside, preferably selected from the group comprising or, alternatively, consisting of: *Lippia citriodora* (Palau) Kunth, *Verbena Officinalis* L. and a mixture thereof, and/or (b) at least one plant of the genus *Plantago* comprising aucubin, preferably selected from the group comprising or, alternatively, consisting of: *Plantago lanceolata* L., *Plantago major* L. and a mixture thereof;
(II) mixing (a) and/or (b) prepared in step (I) with a hydroalcoholic solvent (c), wherein the [(a) and/or (b)]:(c) by weight ratio is comprised in the range from 1:1 to 1:25, preferably from 1:3 to 1:20, more preferably from 1:6 to 1:15 to obtain a mixture of step (II);
(III) heating, under stirring, said mixture of step (II) at a temperature comprised in the range from 40°C to 80°C, preferably from 50°C to 70°C, for a period of time comprised in the range from 20 minutes to 240 minutes, preferably from 30 minutes to 180 minutes, more preferably from 40 minutes to 120 minutes, to obtain a crude extract (d) comprising a solid phase and a liquid phase;
(IV) separating said liquid phase of the crude extract (d) from said solid phase of the crude extract (d) to obtain an isolated liquid phase (e);
(V) concentrating said isolated liquid phase (e), by partially removing the solvent (c) until obtaining a soft extract (f) having a dry residue comprised in the range from 20% to 80% by weight with respect to the weight of the soft extract, preferably from 30% to 70%, more preferably from 40% to 60%;
(VI) drying the soft extract (f) to obtain a dry extract (g), wherein said dry extract (g) has a dry residue at a percentage by weight comprised in the range from 90% to 100% with respect to the weight of the dry extract (g), preferably from 95% to 100%, more preferably from 98% to 100%;

**wherein**
said drying step (VI) is carried out by means of a fluid-bed granulator using a carrier, wherein said carrier is mixed with said soft extract (f) to obtain a dry extract in granular form (h); preferably wherein said carrier is selected from the group comprising or, alternatively, consisting of maltodextrin, gum arabic, inulin, vegetable fibres, polyalcohols, fructooligosaccharides, lecithins, dextrose, isomalt and sugars.

15. The method according to claim 14, wherein

  - in said preparing step (I), the leaves of (a) *Lippia citriodora* and/or leaves of (b) *Plantago lanceolata* are prepared; preferably dried leaves; and/or
  - wherein in said mixing step (II), the hydroalcoholic solvent (c) is an ethanol/water mixture having an alcohol volume comprised in the range from 10° to 80° alcohol; preferably from 20° to 70° alcohol.

**Patentansprüche**

1. Kräuterextrakt, umfassend oder alternativ bestehend aus einem Extrakt von mindestens einer Pflanze der Gattung *Aloysia* und einem Extrakt von mindestens einer Pflanze der Gattung *Plantago,* wobei der Kräuterextrakt Verbascosid in dem Bereich von 3 % bis 10 % und Aucubin in dem Bereich von 0,5 % bis 15 % umfasst, wobei die Prozentsätze Gewichtsprozente in Bezug auf das Gewicht des Kräuterextrakts sind.

2. Kräuterextrakt nach Anspruch 1, wobei die mindestens eine Pflanze der Gattung *Aloysia* ausgewählt ist aus der Gruppe umfassend oder alternativ bestehend aus: *Lippia citriodora* (Palau) Kunth, *Verbena Officinalis* L. und einer Mischung davon; und/oder wobei die mindestens eine Pflanze der Gattung *Plantago* ausgewählt ist aus der Gruppe umfassend oder alternativ bestehend aus: *Plantago lanceolata L., Plantago major* L. und eine Mischung davon.

3. Kräuterextrakt nach Anspruch 1 oder 2, wobei die mindestens eine Pflanze der Gattung *Aloysia Lippia citriodora* (Palau) Kunth ist und wobei die mindestens eine Pflanze der Gattung *Plantago Plantago lanceolata* L. ist.

4. Kräuterextrakt nach einem der Ansprüche 1 bis 3, wobei der Kräuterextrakt Verbascosid in dem Bereich von 3 % bis 10 % und Aucubin in dem Bereich von 1 % bis 5 % umfasst, wobei die Prozentangaben Gewichtsprozente in Bezug auf das Gewicht des Kräuterextrakts sind;
vorzugsweise wobei der Kräuterextrakt ein Trockenextrakt in Granulatform mit einem Trockenrückstand in einem Gewichtsprozentsatz ist, der in dem Bereich von 90 % bis 100 %, bezogen auf das Gesamtgewicht des Trockenextrakts, umfasst ist.

5. Zusammensetzung umfassend oder alternativ bestehend aus: dem Kräuterextrakt nach einem der Ansprüche 1 bis 4 und mindestens einem Zusatzstoff und/oder Hilfsstoff von Lebensmittel- oder pharmazeutischer Qualität.

6. Kräuterextrakt nach einem der Ansprüche 1 bis 4 oder Zusammensetzung nach Anspruch 5 zur Verwendung als Arzneimittel.

7. Kräuterextrakt nach einem der Ansprüche 1 bis 4 oder Zusammensetzung nach Anspruch 5 zur Verwendung bei der symptomatischen Behandlung von entzündlichen Muskel-Skelett-Erkrankungen, rheumatischen Erkrankungen, entzündlichen Gelenkerkrankungen und entzündlichen postoperativen Erkrankungen, vorzugsweise zur Verwendung in Verfahren zur Behandlung von Osteoarthritis, rheumatischer Arthritis und Spondylitis ankylosans.

8. Kräuterextrakt oder Zusammensetzung zur Verwendung nach Anspruch 6 oder 7, wobei der Kräuterextrakt oder die Zusammensetzung auf oralem Weg verabreicht wird.

9. Kräuterextrakt oder Zusammensetzung zur Verwendung nach Anspruch 7 oder 8, wobei der Kräuterextrakt oder die Zusammensetzung zur Verwendung bei der vorbeugenden und/oder heilenden Behandlung von Osteoarthritis des Knies, der Hüfte, der Schulter, der Hand und/oder der Halswirbelsäule ist.

10. Kräuterextrakt oder Zusammensetzung zur Verwendung nach Anspruch 9, wobei der Kräuterextrakt oder die Zusammensetzung zur Verwendung bei der vorbeugenden und/oder heilenden Behandlung von Osteoarthritis des Knies ist.

11. Kräuterextrakt nach einem der Ansprüche 1 bis 4 oder Zusammensetzung nach Anspruch 5 zur Verwendung als analgetisches und/oder antispasmoisches Arzneimittel durch Verabreichen einer therapeutisch wirksamen Menge des Kräuterextrakts oder der Zusammensetzung an ein bedürftiges Subjekt, vorzugsweise auf oralem Weg.

12. Kräuterextrakt oder Zusammensetzung zur Verwendung nach Anspruch 11, wobei der Kräuterextrakt oder die Zusammensetzung zur Verwendung in einem Verfahren zur Behandlung von Zahnschmerzen, Dysmenorrhoe, postoperativen Schmerzen, Halswirbelsäulenschmerzen, arthritischen Schmerzen, Unterleibsschmerzen oder -krämpfen, Muskel-Skelett-Schmerzen, Schmerzen nach der Geburt, sportbedingten Traumata oder Prellungsschmerzen und/oder allgemeinen Schmerzen ist.

13. Kräuterextrakt oder Zusammensetzung zur Verwendung nach einem der Ansprüche 7 bis 12, wobei der Kräuterextrakt oder die Zusammensetzung dem bedürftigen Subjekt auf oralem Weg in einer solchen Menge verabreicht wird, dass der Kräuterextrakt in einer Menge bereitgestellt wird, die in dem Bereich von 400 mg/Tag bis 1600 mg/Tag, vorzugsweise von 500 mg/Tag bis 1200 mg/Tag, besonders bevorzugt von 600 mg/Tag bis 1000 mg/Tag umfasst ist.

14. Verfahren zum Herstellen des Kräuterextrakts nach einem der Ansprüche 1 bis 4, umfassend die folgenden Schritte:

    (I) Herstellen - in einer Extraktionsanlage - von (a) mindestens einer Pflanze der Gattung *Aloysia* umfassend Verbascosid vorzugsweise ausgewählt aus der Gruppe umfassend oder alternativ bestehend aus: *Lippia citriodora* (Palau) Kunth, *Verbena Officinalis* L. und eine Mischung davon, und/oder (b) mindestens eine Pflanze der Gattung *Plantago* umfassend Aucubin, vorzugsweise ausgewählt aus der Gruppe umfassend oder alternativ bestehend aus: *Plantago lanceolata L., Plantago major* L. und eine Mischung davon;
    (II) Mischen von (a) und/oder (b), hergestellt in Schritt (I), mit einem hydroalkoholischen Lösungsmittel (c), wobei das [(a) und/oder (b)]: (c) in einem Gewichtsverhältnis in dem Bereich von 1:1 bis 1:25, vorzugsweise von 1:3 bis 1:20, besonders bevorzugt von 1:6 bis 1:15 umfasst ist, um eine Mischung aus Schritt (II) zu erhalten;
    (III) Erhitzen, unter Rühren, der Mischung von Schritt (II) auf eine Temperatur, die in dem Bereich von 40 °C bis 80 °C, vorzugsweise von 50 °C bis 70 °C, umfasst ist, für einen Zeitraum, der in dem Bereich von 20 Minuten bis 240 Minuten, vorzugsweise von 30 Minuten bis 180 Minuten, besonders bevorzugt von 40 Minuten bis 120 Minuten, umfasst ist, um einen Rohextrakt (d) zu erhalten, der eine feste Phase und eine flüssige Phase umfasst;
    (IV) Abtrennen der flüssigen Phase des Rohextrakts (d) von der festen Phase des Rohextrakts (d), um eine isolierte flüssige Phase (e) zu erhalten;
    (V) Konzentrieren der isolierten flüssigen Phase (e) durch teilweises Entfernen des Lösungsmittels (c) bis zum Erhalten eines Weichextrakts (f) mit einem Trockenrückstand, der in dem Bereich von 20 bis 80 Gew.-%, bezogen auf das Gewicht des Weichextrakts, umfasst ist, vorzugsweise von 30 bis 70 %, besonders bevorzugt von 40 % bis 60 %;
    (VI) Trocknen des Weichextrakts (f), um einen Trockenextrakt (g) zu erhalten, wobei der Trockenextrakt (g) einen Trockenrückstand in einem Gewichtsprozentsatz aufweist, der in dem Bereich von 90 % bis 100 %, bezogen auf das Gewicht des Trockenextrakts (g), umfasst ist, vorzugsweise von 95 % bis 100 %, besonders bevorzugt von 98 % bis 100 %;

    **wobei**
    der Schritt des Trocknens (VI) mittels eines Wirbelschichtgranulators unter Verwendung eines Trägers durchgeführt wird, wobei der Träger mit dem Weichextrakt (f) gemischt wird, um einen Trockenextrakt in Granulatform (h) zu erhalten; vorzugsweise wobei der Träger ausgewählt ist aus der Gruppe umfassend oder alternativ bestehend aus Maltodextrin, Gummi arabicum, Inulin, pflanzlichen Fasern, Polyalkoholen, Fructooligosacchariden, Lecithinen, Dextrose, Isomalt und Zuckern.

15. Verfahren nach Anspruch 14, wobei

    - in dem Schritt des Herstellens (I) die Blätter von (a) *Lippia citriodora* und/oder Blätter von (b) *Plantago lanceolata* hergestellt werden; vorzugsweise getrocknete Blätter; und/oder
    - wobei in dem Schritt des Mischens (II) das hydroalkoholische Lösungsmittel (c) eine Ethanol/Wasser-Mischung mit einem Alkoholvolumen ist, das in dem Bereich von 10° bis 80° Alkohol umfasst ist; vorzugsweise von 20° bis 70° Alkohol.


**Revendications**

1. Extrait végétal comprenant ou, en variante, consistant en un extrait d'au moins une plante du genre *Aloysia* et un extrait d'au moins une plante du genre *Plantago,* dans lequel ledit extrait végétal comprend du verbascoside dans la plage de 3 % à 10 % et de l'aucubine dans la plage de 0,5 % à 15 %, dans lequel lesdits pourcentages sont des pourcentages en poids par rapport au poids dudit extrait végétal.

**2.** Extrait végétal selon la revendication 1, dans lequel ladite au moins une plante du genre *Aloysia* est choisie dans le groupe comprenant ou, en variante, consistant en : *Lippia citriodora* (Palau) Kunth, *Verbena Officinalis* L. et un mélange de ceux-ci ; et/ou dans lequel ladite au moins une plante du genre *Plantago* est choisie dans le groupe comprenant ou, alternativement, consistant en : *Plantago lanceolata L., Plantago major* L. et un mélange de ceux-ci.

**3.** Extrait végétal selon la revendication 1 ou 2, dans lequel ladite au moins une plante du genre *Aloysia* est *Lippia citriodora* (Palau) Kunth et dans lequel ladite au moins une plante du genre *Plantago* est *Plantago lanceolata L.*

**4.** Extrait végétal selon l'une quelconque des revendications 1 à 3, dans lequel ledit extrait végétal comprend du verbascoside dans la plage de 3 % à 10 % et de l'aucubine dans la plage de 1 % à 5 %, dans lequel lesdits pourcentages sont des pourcentages en poids par rapport au poids dudit extrait végétal ;
de préférence dans lequel ledit extrait végétal est un extrait sec sous forme granulaire ayant un résidu sec à un pourcentage en poids compris entre 90 % et 100 % par rapport au poids total de l'extrait sec.

**5.** Composition comprenant ou, en variante, consistant en : ledit extrait végétal selon l'une quelconque des revendications 1 à 4, et au moins un additif et/ou excipient de qualité alimentaire ou pharmaceutique.

**6.** Extrait végétal selon l'une quelconque des revendications 1 à 4 ou composition selon la revendication 5 destiné à être utilisé en tant que médicament.

**7.** Extrait végétal selon l'une quelconque des revendications 1 à 4 ou composition selon la revendication 5 destiné à être utilisé dans le traitement symptomatique des maladies musculo-squelettiques inflammatoires, des maladies rhumatoïdes, des maladies articulaires inflammatoires et des maladies postopératoires inflammatoires, de préférence destiné à être utilisé dans des procédés de traitement de l'ostéoarthrose, de la polyarthrite rhumatoïde et de la spondylarthrite ankylosante.

**8.** Extrait végétal ou composition destiné à être utilisé selon la revendication 6 ou 7, dans lequel ledit extrait végétal ou composition est administré par voie orale.

**9.** Extrait végétal ou composition destiné à être utilisé selon la revendication 7 ou 8, dans lequel ledit extrait végétal ou composition est destiné à être utilisé dans le traitement préventif et/ou curatif de l'ostéoarthrose du genou, de la hanche, de l'épaule, de la main et/ou des cervicales.

**10.** Extrait végétal ou composition destiné à être utilisé selon la revendication 9, dans lequel ledit extrait végétal ou ladite composition est destiné à être utilisé dans le traitement préventif et/ou curatif de l'ostéoarthrose du genou.

**11.** Extrait végétal selon l'une quelconque des revendications 1 à 4 ou composition selon la revendication 5 destiné à être utilisé en tant que médicament analgésique et/ou antispasmotique, par administration d'une quantité thérapeutiquement efficace dudit extrait végétal ou composition à un sujet nécessiteux, de préférence par voie orale.

**12.** Extrait végétal ou composition destiné à être utilisé selon la revendication 11, dans lequel ledit extrait végétal ou ladite composition est destiné à être utilisé dans un procédé pour le traitement des maux de dents, de la dysménorrhée, de la douleur postopératoire, de la douleur cervicale, de la douleur arthritique, de la douleur abdominale ou des crampes, de la douleur musculo-squelettique, de la douleur post-partum, du traumatisme lié au sport ou de la douleur de contusion et/ou de la douleur générale.

**13.** Extrait végétal ou composition pour utilisation selon l'une quelconque des revendications 7 à 12, dans lequel ledit extrait végétal ou ladite composition est administré par voie orale au sujet nécessiteux en une quantité telle qu'elle fournit ledit extrait végétal en une quantité comprise dans la plage de 400 mg/jour à 1600 mg/jour, de préférence de 500 mg/jour à 1200 mg/jour, plus préférablement de 600 mg/jour à 1000 mg/jour.

**14.** Procédé de préparation de l'extrait végétal selon l'une quelconque des revendications 1 à 4, comprenant les étapes consistant à :

(I) préparer - dans une usine d'extraction - (a) au moins une plante du *genre Aloysia* comprenant du verbascoside, de préférence choisi dans le groupe comprenant ou, alternativement, consistant en : *Lippia citriodora* (Palau) Kunth, *Verbena Officinalis* L. et un mélange de ceux-ci, et/ou (b) au moins une plante du genre *Plantago* comprenant de l'aucubine, de préférence choisie dans le groupe comprenant ou, alternativement, consistant *en :*

*Plantago lanceolata* L., *Plantago major* L. et un mélange de ceux-ci ;

(II) mélanger (a) et/ou (b) préparés à l'étape (I) avec un solvant hydroalcoolique (c), dans lequel [(a) et/ou (b)] : (c) par rapport en poids est compris dans la plage de 1:1 à 1:25, de préférence de 1:3 à 1:20, plus préférablement de 1:6 à 1:15 pour obtenir un mélange de l'étape (II) ;

(III) chauffer, sous agitation, ledit mélange de l'étape (II) à une température comprise dans la plage de 40°C à 80°C, de préférence de 50°C à 70°C, pendant une période de temps comprise dans la plage de 20 minutes à 240 minutes, de préférence de 30 minutes à 180 minutes, plus préférablement de 40 minutes à 120 minutes, pour obtenir un extrait brut (d) comprenant une phase solide et une phase liquide ;

(IV) séparer ladite phase liquide de l'extrait brut (d) de ladite phase solide de l'extrait brut (d) pour obtenir une phase liquide isolée (e) ;

(V) concentrer ladite phase liquide isolée (e), en éliminant partiellement le solvant (c) jusqu'à l'obtention d'un extrait mou (f) ayant un résidu sec compris dans la plage de 20 % à 80 % en poids par rapport au poids de l'extrait mou, de préférence de 30 % à 70 %, plus préférablement de 40 % à 60 % ;

(VI) sécher l'extrait mou (f) pour obtenir un extrait sec (g), dans lequel ledit extrait sec (g) a un résidu sec à un pourcentage en poids compris dans la plage de 90 % à 100 % par rapport au poids de l'extrait sec (g), de préférence de 95 % à 100 %, plus préférablement de 98 % à 100 % ;

**dans lequel**

ladite étape de séchage (VI) est réalisée au moyen d'un granulateur à lit fluidisé utilisant un support, dans lequel ledit support est mélangé avec ledit extrait mou (f) pour obtenir un extrait sec sous forme granulaire (h) ; de préférence dans lequel ledit support est choisi dans le groupe comprenant ou, en variante, consistant en la maltodextrine, la gomme arabique, l'inuline, les fibres végétales, les polyalcools, les fructooligosaccharides, les lécithines, le dextrose, l'isomalt et les sucres.

15. Procédé selon la revendication 14, dans lequel

- dans ladite étape de préparation (I), les feuilles de (a) *Lippia citriodora* et/ou les feuilles de (b) *Plantago lanceolata* sont préparées ; de préférence des feuilles séchées ; et/ou
- dans lequel dans ladite étape de mélange (II), le solvant hydroalcoolique (c) est un mélange éthanol/eau ayant un volume d'alcool compris dans la plage de 10° à 80° alcool ; de préférence de 20° à 70° alcool.

Figure 1

Figure 2

Figure 3

Figure 4

Figure 5

Figure 6

DAD1 A, Sig=205,4 Ref=360,100 (AUCUBINA STD 02.D)

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004069218 A1
- US 2005106113 A1

**Non-patent literature cited in the description**

- **SANCHEZ**. *In vivo anti-inflammatory and anti-ulcerogenic activities of extracts from wild growing and in vitro plants of Castilleja tenuiflora Benth (Orobanchaceae)*
- **GEORGIEV**. *Verbascum xanthophoeniceum-derived phenylethanoid glycosides are potent inhibitors of inflammatory chemokines in dormant and interferon-gamma-stimulated human keratinocytes*
- **GEORGIEV M**. *J. of Ethnopharmacology*, 01 February 2013, 754-760
- **SANCHEZ PAUL MAURICIO et al.** *J. of Ethnopharmacology*, vol. 150 (3), 1032-1037
- **FRANZYK**. *A caffeoyl phenylethanoid glycoside from Plantago myosuros*
- *CHEMICAL ABSTRACTS*, 61276-17-3
- *CHEMICAL ABSTRACTS*, 479-98-1
- *Italian Legislative Decree*, 24 February 1997 (46)
- **AGRAWAL S. et al.** Solubility enhancement of poorly water-soluble Celecoxib for parenteral formulations.. *Int J Pharm Sei*, 2012, vol. 3, 2325-2336
- **SALAFFI et al.** *Osteoarthritis cartilage*, 2003, vol. 11, 551-60